# EUROPEAN PATENT APPLICATION

(11) **EP 3 991 745 A1**
(43) Date of publication of application: **04.05.2022**
(21) Application number: 20833335.1
(22) Date of filing: 24.06.2020
(51) Int. Cl.: A61K 39/395, C07K 16/28, C12N 15/13, C12N 15/63, C12N 5/10, A61P 35/00

(54) **FORMULATIONS CONTAINING ANTI-CD47/PD-L1 BISPECIFIC ANTIBODY AND PREPARATION METHOD THEREFOR AND USE THEREOF**

(30) Priority: 25.06.2019 CN 201910554231; 16.06.2020 CN 202010550660
(71) Applicant: Innovent Biologics (Suzhou) Co., Ltd., Suzhou, Jiangsu 215123 (CN)
(72) Inventor: ZHU, Xinggui, Suzhou, Jiangsu 215123 (CN); MA, Yidong, Suzhou, Jiangsu 215123 (CN); WANG, Yinjue, Suzhou, Jiangsu 215123 (CN); ZHOU, Kaisong, Suzhou, Jiangsu 215123 (CN)
(74) Representative: Ruscoe, Peter James
(86) International application number: PCT/CN2020/098172
(87) International publication number: WO 2020/259605

(57) **Abstract**

The present invention relates to formulations comprising an anti-CD47/PD-L1 bispecific antibody, and in particular to pharmaceutical formulations comprising an anti-CD47/PD-L1 bispecific antibody, a buffer, a stabilizer and a surfactant. Furthermore, the present invention also relates to therapeutic or prophylactic use of these formulations.

## Description

### TECHNICAL FIELD

The present invention relates to the field of antibody formulations. Specifically, the present invention relates to a pharmaceutical formulation comprising a recombinant anti-cluster of differentiation 47 (CD47) and anti-programmed death ligand 1 (PD-L1) bispecific antibody (also referred to as anti-CD47/PD-L1 bispecific antibody), and in particular to a stable high-concentration antibody liquid formulation, a method for preparing the pharmaceutical formulation, and therapeutic and/or prophylactic use of the pharmaceutical formulation.

### BACKGROUND

PD-L1, also known as cluster of differentiation 274 (CD274) or B7 homolog 1 (B7-H1), is a 40-kDa type I transmembrane protein. PD-L1 binds to the receptor PD-1 thereof present on activated T cells and down-regulates T cell activation (Latchman et al., 2001, Nat. Immunol., 2:261-8; Carter et al., 2002, Eur. J. Immunol., 32:634-43). PD-L1 expression has been found in many cancers, including human lung cancer, ovarian cancer, colon cancer, and several myelomas, and PD-L1 expression is often associated with poor prognosis of cancers (Iwai et al., (2002) PNAS, 99:12293-7; Ohigashi et al., (2005) Clin Cancer Res., 11:2947-53; Okazaki et al., (2007) Intern. Immun., 19:813-24; Thompson et al., (2006) Cancer Res., 66:3381-5). It has been proposed that, in some patients with tumors, immunosuppression can be reversed by suppressing local interactions between PD1 and PD-L1.

The anti-PD-L1 antibody atezolizumab developed by Roche, the anti-PD-L1 antibody avelumab jointly developed by Merck KGaA and Pfizer, and durvalumab developed by AstraZeneca have shown their efficacy in treating some patients with tumors. Other anti-PD-L1 antibodies include YW243.55.S70 (the heavy and light chain variable regions are set forth in SEQ ID NOs: 20 and 21 in WO2010/077634), the anti-PD-L1 antibody disclosed in WO2007/005874, and so on.

Cluster of differentiation 47 (CD47), also known as an integrin-associated protein (IAP), is a member of the immunoglobulin superfamily. CD47 interacts with SIRPα, a cell surface immunoglobulin as its ligand mainly expressed by macrophages and dendritic cells, producing a series of cascade reactions, and thereby inhibiting the uptake and phagocytosis of cells expressing CD47 by macrophages and dendritic cells. CD47 overexpression has been observed in tumors. However, the expression of CD47 is also found in many normal tissues, which leads to non-specific binding of antibodies targeting CD47 only to normal blood system cells and thus causes antigen sink.

The anti-CD47/PD-L1 bispecific antibody simultaneously targets CD47 and PD-L1 on tumor cells. The specific binding to PD-L1 on tumor cells promotes the selective binding of the anti-CD47/PD-L1 bispecific antibody to tumor cells and avoids the binding to CD47 expressed in many normal tissues. Thus, the anti-CD47/PD-L1 bispecific antibody can reduce side effects while enhancing anti-tumor effect.

PCT application No. PCT/CN2018/123886 (filed on Dec. 26, 2018) discloses a novel antibody model, and constructs and expresses an anti-CD47/PD-L1 bispecific antibody with the novel antibody model. The anti-CD47/PD-L1 bispecific antibody is administered to tumor-bearing mice obtained by inoculating NOD-SCID mice with Raji-PD-L1 cells, and results show that compared with the administration of an anti-CD47 monoclonal antibody and an anti-PD-L1 monoclonal antibody, the anti-CD47/PD-L1 bispecific antibody has significantly improved anti-tumor activity, can remarkably inhibit the growth of tumors and even can completely eliminate the tumors. Furthermore, the anti-CD47/PD-L1 bispecific antibody also shows significantly reduced hemagglutination and thus will have significantly reduced side effects in clinical treatment. There is a need in the art for an anti-CD47/PD-L1 bispecific antibody formulation that can be used to treat, prevent or delay a variety of diseases associated with the SIRPα/CD47 signaling pathway and the PD1/PD-L1 signaling pathway.

The antibody formulation needs to be formulated in a manner that the antibody is made suitable for administration to a subject and in a manner that the antibody maintains stable in storage and subsequent use as well. For example, if an antibody is not properly formulated in a liquid, the antibody in the liquid solution is prone to decomposition, aggregation, undesirable chemical modification or the like. The stability of an antibody in an antibody formulation depends on the buffer, stabilizer, surfactant and the like used in the formulation.

Although some anti-CD47/PD-L1 bispecific antibodies are known, there remains a need in the art for novel pharmaceutical formulations comprising an anti-CD47/PD-L1 bispecific antibody that are sufficiently stable and suitable for administration to a subject. Thus, there is a need for a suitable anti-CD47/PD-L1 bispecific antibody formulation for use in treating or preventing diseases.

### BRIEF SUMMARY

The present invention satisfies the above-described need by providing a pharmaceutical formulation comprising an anti-CD47/PD-L1 bispecific antibody protein specifically binding to CD47 and PD-L1.

In one aspect, the present invention provides a liquid antibody formulation comprising (i) an anti-CD47/PD-L1 bispecific antibody protein; (ii) a buffer; (iii) a stabilizer; and (iv) a surfactant.

The anti-CD47/PD-L1 bispecific antibody protein comprised in the antibody formulation disclosed herein is a triple-chain antibody, which comprises a VH/VL pair specifically binding to CD47 on a first polypeptide chain and a second polypeptide chain as a first antigen-binding site, and a first VHH and a second VHH specifically binding to PD-L1 on a third polypeptide chain as a second single domain antigen-binding site and a third single domain antigen-binding site, respectively; or comprises a VH/VL pair specifically binding to PD-L1 on a first polypeptide chain and a second polypeptide chain as a first antigen-binding site, and a first VHH and a second VHH specifically binding to CD47 on a third polypeptide chain as a second single domain antigen-binding site and a third single domain antigen-binding site, respectively. In some embodiments, the anti-CD47/PD-L1 bispecific antibody protein is capable of binding to CD47 on the surface of tumor cells with an affinity constant of at least about 10⁷ M⁻¹, preferably about 10⁸ M⁻¹, and more preferably about 10⁹ M⁻¹ or greater, thereby blocking the binding of CD47 to SIRPα on the surface of macrophages and promoting the phagocytosis of tumor cells by macrophages in the tumor tissue infiltration area; and capable of binding to PD-L1 on the surface of tumor cells with an affinity constant of at least about 10⁷ M⁻¹, preferably about 10⁸ M⁻¹, and more preferably about 10⁹ M⁻¹ or greater, thereby inhibiting the binding of PD-1 on T cells to PD-L1 on the surface of tumor cells, inducing T cell activation and exerting anti-tumor effect.

In one embodiment, the anti-CD47/PD-L1 bispecific antibody protein is the recombinant anti-CD47/PD-L1 bispecific antibody protein disclosed in PCT application No. PCT/CN2018/123886 (filed on Dec. 26, 2018), the content of which is incorporated herein by reference in its entirety for the purpose of the present application. In one embodiment, the anti-CD47/PD-L1 bispecific antibody protein is a triple-chain antibody, which comprises a VH/VL pair specifically binding to CD47 on a first polypeptide chain and a second polypeptide chain as a first antigen-binding site, and a first VHH and a second VHH specifically binding to PD-L1 on a third polypeptide chain as a second single domain antigen-binding site and a third single domain antigen-binding site, respectively, wherein the VH/VL pair specifically binding to CD47 on the first polypeptide chain and the second polypeptide chain, as the first antigen-binding site, comprises a VH CDR1 set forth in GSIEHYYWS (SEQ ID NO: 3), a VH CDR2 set forth in YIYYSGSTNYNPSLKS (SEQ ID NO: 4), a VH CDR3 set forth in ARGKTGSAA (SEQ ID NO: 5), a VL CDR1 set forth in RASQGISRWLA (SEQ ID NO: 10), a VL CDR2 set forth in AASSLQS (SEQ ID NO: 11) and a VL CDR3 set forth in QQTVSFPIT (SEQ ID NO: 12) derived from anti-CD47 antibody ADI-29341, or sequences having one, two, three, four, five, six or more amino acid changes (e.g., amino acid substitutions or deletions) compared with one or more CDRs of the 6 CDRs; the second single domain antigen-binding site and the third single domain antigen-binding site specifically binding to PD-L1 on the third polypeptide chain both comprise a CDR1 set forth in AYTISRNSMG (SEQ ID NO: 17), a CDR2 set forth in IESDGST (SEQ ID NO: 18) and a CDR3 set forth in AAPKVGLGPRTALGHLAFMTLPALNY (SEQ ID NO: 19), or sequences having one, two, three, four, five, six or more amino acid changes (e.g., amino acid substitutions or deletions) compared with one or more CDRs of the 3 CDRs.

In one embodiment, the VH/VL pair specifically binding to CD47 on the first polypeptide chain and the second polypeptide chain of the anti-CD47/PD-L1 bispecific antibody protein, as the first antigen-binding site, comprises paired heavy chain variable region/light chain variable region sequences of SEQ ID NOs: 2/9 derived from anti-CD47 antibody ADI-29341, or sequences having at least 90%, 91%, 92%, 93%, 94%, 95%, 96%, 97%, 98%, 99% or higher sequence identity to the paired heavy chain variable region/light chain variable region sequences, and the second single domain antigen-binding site and the third single domain antigen-binding site specifically binding to PD-L1 on the third polypeptide chain both comprise sequences set forth in SEQ ID NO: 15 and/or SEQ ID NO: 16, or sequences substantially identical (e.g., having at least 80%, 85%, 90%, 92%, 95%, 97%, 98%, 99% or higher identity) thereto:

QVQLQESGPGLVKPSETLSLTCTVSGGSIEHYYWSWIRQPPGKGLEWIGYIYYSGSTNYNPS
LKSRVTISVDTSKNQFSLKLSSVTAADTAVYYCARGKTGSAAWGQGTLVTVSS (SEQ ID
NO: 2);

DIQMTQSPSSVSASVGDRVTITCRASQGISRWLAWYQQKPGKAPKLLIYAASSLQSGVPSRF
SGSGSGTDFTLTISSLQPEDFATYYCQQTVSFPITFGGGTKVEIK (SEQ ID NO: 9);

QVQLQESGGGSVQAGGSLRLSCQASAYTISRNSMGWFRQAPGKQREGVAAIESDGSTSYS
DSVKGRFTISLGNAKNTLYLEMNSLKPEDTAMYYCAAPKVGLGPRTALGHLAFMTLPALN
YWGQGTQVTVSS (SEQ ID NO: 15);

QVQLQESGGGLVQPGGSLRLSCAASAYTISRNSMGWFRQAPGKGLEGVAAIESDGSTSYS
DSVKGRFTISLDNSKNTLYLEMNSLRAEDTAVYYCAAPKVGLGPRTALGHLAFMTLPALN
YWGQGTLVTVSS (SEQ ID NO: 16).

In one embodiment, the anti-CD47/PD-L1 bispecific antibody protein comprises a first polypeptide chain set forth in SEQ ID NO: 1, a second polypeptide chain set forth in SEQ ID NO: 8, and a third polypeptide chain set forth in SEQ ID NO: 14 or SEQ ID NO: 22, or sequences substantially identical (e.g., having at least 80%, 85%, 90%, 92%, 95%, 97%, 98%, 99% or higher identity) to any one of the sequences.

QVQLQESGPGLVKPSETLSLTCTVSGGSIEHYYWSWIRQPPGKGLEWIGYIYYSGSTNYNPS
LKSRVTISVDTSKNQFSLKLSSVTAADTAVYYCARGKTGSAAWGQGTLVTVSSASTKGPS
VFPLAPSSKSTSGGTAALGCLVKDYFPEPVTVSWNSGALTSGVHTFPAVLQSSGLYSLSSV
VTVPSSSLGTQTYICNVNHKPSNTKVDKKVEPKSCDKTHTCPPCPAPEAAGGPSVFLFPPKP
KDTLMISRTPEVTCVVVDVSHEDPEVKFNWYVDGVEVHNAKTKPREEQYNSTYRVVSVL
TVLHQDWLNGKEYKCKVSNKALPAPIEKTISKAKGQPREPQVYTLPPCRDELTKNQVSLW
CLVKGFYPSDIAVEWESNGQPENNYKTTPPVLDSDGSFFLYSKLTVDKSRWQQGNVFSCS
VMHEALHNHYTQKSLSLSPG (SEQ ID NO: 1);

DIQMTQSPSSVSASVGDRVTITCRASQGISRWLAWYQQKPGKAPKLLIYAASSLQSGVPSRF
SGSGSGTDFTLTISSLQPEDFATYYCQQTVSFPITFGGGTKVEIKRTVAAPSVFIFPPSDEQLK
SGTASVVCLLNNFYPREAKVQWKVDNALQSGNSQESVTEQDSKDSTYSLSSTLTLSKADY
EKHKVYACEVTHQGLSSPVTKSFNRGEC (SEQ ID NO: 8);

QVQLQESGGGLVQPGGSLRLSCAASAYTISRNSMGWFRQAPGKGLEGVAAIESDGSTSYS
DSVKGRFTISLDNSKNTLYLEMNSLRAEDTAVYYCAAPKVGLGPRTALGHLAFMTLPALN
YWGQGTLVTVSSGGGGSGGGGSGGGGSGGGGSQVQLQESGGGLVQPGGSLRLSCAASAY
TISRNSMGWFRQAPGKGLEGVAAIESDGSTSYSDSVKGRFTISLDNSKNTLYLEMNSLRAE
DTAVYYCAAPKVGLGPRTALGHLAFMTLPALNYWGQGTLVTVSSDKTHTCPPCPAPEAA
GGPSVFLFPPKPKDTLMISRTPEVTCVVVDVSHEDPEVKFNWYVDGVEVHNAKTKPREEQ
YNSTYRVVSVLTVLHQDWLNGKEYKCKVSNKALPAPIEKTISKAKGQPREPQVCTLPPSRD
ELTKNQVSLSCAVKGFYPSDIAVEWESNGQPENNYKTTPPVLDSDGSFFLVSKLTVDKSRW
QQGNVFSCSVMHEALHNHYTQKSLSLSPG (SEQ ID NO: 14);

QVQLQESGGGLVQPGGSLRLSCAASAYTISRNSMGWFRQAPGKGLEGVAAIESDGSTSYS
DSVKGRFTISLDNSKNTLYLEMNSLRAEDTAVYYCAAPKVGLGPRTALGHLAFMTLPALN
YWGQGTLVTVSSQVQLQESGGGLVQPGGSLRLSCAASAYTISRNSMGWFRQAPGKGLEG
VAAIESDGSTSYSDSVKGRFTISLDNSKNTLYLEMNSLRAEDTAVYYCAAPKVGLGPRTAL
GHLAFMTLPALNYWGQGTLVTVSSDKTHTCPPCPAPEAAGGPSVFLFPPKPKDTLMISRTP
EVTCVVVDVSHEDPEVKFNWYVDGVEVHNAKTKPREEQYNSTYRVVSVLTVLHQDWLN
GKEYKCKVSNKALPAPIEKTISKAKGQPREPQVCTLPPSRDELTKNQVSLSCAVKGFYPSDI
AVEWESNGQPENNYKTTPPVLDSDGSFFLVSKLTVDKSRWQQGNVFSCSVMHEALHNHY
TQKSLSLSPG (SEQ ID NO: 22).

In one embodiment, the anti-CD47/PD-L1 bispecific antibody protein is an anti-CD47/PD-L1 bispecific antibody protein recombinantly expressed in HEK293 cells or CHO cells.

In one embodiment, the concentration of the anti-CD47/PD-L1 bispecific antibody protein in the liquid antibody formulation disclosed herein is about 1-200 mg/mL. In another embodiment, the concentration of the anti-CD47/PD-L1 bispecific antibody protein in the liquid antibody formulation disclosed herein is about 5-150 mg/mL. In other embodiments, the concentration of the anti-CD47/PD-L1 bispecific antibody protein in the liquid antibody formulation disclosed herein is about 5, 10, 20, 30, 40, 50, 60, 70, 80, 90, 100, 110, 120, 130, 140, or 150 mg/mL.

In one embodiment, the concentration of the buffer in the liquid antibody formulation disclosed herein is about 1-30 mM. In one embodiment, the concentration of the buffer in the liquid antibody formulation disclosed herein is about 5-25 mM, e.g., about 5, 10, 15, 20 or 25 mM.

In one embodiment, the buffer is selected from histidine, histidine hydrochloride and a combination thereof.

In one embodiment, the concentration of the stabilizer in the liquid antibody formulation disclosed herein is about 50-500 mM. In one embodiment, the concentration of the stabilizer in the liquid antibody formulation disclosed herein is about 100-400 mM, e.g., about 100, 150, 200, 250, 300, 350 or 400 mM.

In one embodiment, the stabilizer is selected from sorbitol, sucrose, trehalose, arginine, arginine hydrochloride and any combination thereof, and is preferably sucrose, arginine and/or arginine hydrochloride. In one embodiment, the liquid antibody formulation comprises arginine hydrochloride as the stabilizer; arginine hydrochloride is preferably present in an amount of about 50-250 mM, and more preferably about 100-200 mM (e.g., about 100, 110, 120, 130, 140, 150, 160, 170, 180, 190 or 200 mM); and/or the liquid antibody formulation comprises sucrose as the stabilizer; sucrose is preferably present in an amount of about 50-250 mM, and more preferably about 100-200 mM (e.g., about 100, 110, 120, 130, 140, 150, 160, 170, 180, 190 or 200 mM).

In one embodiment, the concentration of the surfactant in the liquid antibody formulation disclosed herein is about 0.1-1 mg/mL. In one embodiment, the concentration of the surfactant in the liquid antibody formulation disclosed herein is about 0.2-0.8 mg/mL, e.g., about 0.2, 0.3, 0.4, 0.5, 0.6, 0.7 or 0.8 mg/mL.

In one embodiment, the surfactant is a nonionic surfactant. In one embodiment, the surfactant is selected from polysorbate surfactants. In one specific embodiment, the surfactant in the liquid antibody formulation disclosed herein is polysorbate-80.

In some embodiments, the liquid formulation further comprises a metal chelating agent (e.g., EDTA), for example, about 0.002-0.2 mg/mL metal chelating agent (e.g., EDTA). In one embodiment, the liquid formulation further comprises about 0.01-0.1 mg/mL, e.g., about 0.01, 0.02, 0.03, 0.04, 0.05, 0.06, 0.08 or 0.1 mg/mL, metal chelating agent (e.g., EDTA).

In one embodiment, the pH of the liquid formulation is about 6.4-7.0. In some embodiments, the pH of the liquid formulation is any of about 6.4-7.0, e.g., about 6.4, 6.5, 6.6, 6.7, 6.8, 6.9 or 7.0.

In one embodiment, the liquid formulation is a pharmaceutical formulation, preferably an injection, and more preferably a subcutaneous injection or an intravenous injection. In one embodiment, the liquid formulation is an intravenous infusion.

In one embodiment, the liquid antibody formulation disclosed herein comprises:
(i) about 1-200 mg/mL anti-CD47/PD-L1 bispecific antibody protein;
(ii) about 1-30 mM histidine and/or histidine hydrochloride;
(iii) about 50-500 mM sucrose, arginine and/or arginine hydrochloride; and
(iv) about 0.1-1 mg/mL polysorbate-80, wherein
optionally, the liquid formulation further comprises 0.002-0.2 mg/mL metal chelating agent (e.g., EDTA), wherein
the pH of the liquid formulation is about 6.4-7.0, preferably about 6.5.

In one preferred embodiment, the liquid antibody formulation disclosed herein comprises:
(i) about 50-150 mg/mL anti-CD47/PD-L1 bispecific antibody protein;
(ii) about 3-25 mM histidine and/or histidine hydrochloride;
(iii) about 150-300 mM sucrose, arginine and/or arginine hydrochloride; and
(iv) about 0.2-0.8 mg/mL polysorbate-80, wherein
optionally, the liquid formulation further comprises about 0.01-0.1 mg/mL metal chelating agent (e.g., EDTA), wherein
the pH of the liquid formulation is about 6.4-7.0, preferably about 6.5.

In one preferred embodiment, the liquid antibody formulation disclosed herein comprises:
(i) about 100 mg/mL anti-CD47/PD-L1 bispecific antibody protein;
(ii) about 20 mM histidine;
(iii) about 180 mM arginine hydrochloride; and
(iv) about 0.2 mg/mL polysorbate-80, wherein
optionally, the liquid formulation further comprises a metal chelating agent (e.g., EDTA), e.g., about 0.02 mg/mL EDTA, wherein
the pH of the liquid formulation is about 6.4-7.0, preferably about 6.5.

In one preferred embodiment, the liquid antibody formulation disclosed herein comprises:
(i) about 100 mg/mL anti-CD47/PD-L1 bispecific antibody protein;
(ii) about 20 mM histidine;
(iii) about 100 mM arginine hydrochloride and 4% sucrose; and
(iv) about 0.2 mg/mL polysorbate-80, wherein
optionally, the liquid formulation further comprises a metal chelating agent (e.g., EDTA), e.g., about 0.02 mg/mL EDTA, wherein
the pH of the liquid formulation is about 6.4-7.0, preferably about 6.5.

In one preferred embodiment, the liquid antibody formulation disclosed herein comprises:
(i) about 100 mg/mL anti-CD47/PD-L1 bispecific antibody protein;
(ii) about 2.52 mg/mL histidine and about 0.79 mg/mL histidine hydrochloride;
(iii) about 37.92 mg/mL arginine hydrochloride; and
(iv) about 0.5 mg/mL polysorbate-80, wherein
optionally, the liquid formulation further comprises a metal chelating agent (e.g., EDTA), e.g., about 0.02 mg/mL EDTA, wherein
the pH of the liquid formulation is about 6.4-7.0, preferably about 6.5.

In another aspect, the present invention provides a solid antibody formulation obtained by solidifying the liquid antibody formulation disclosed herein. The solidification treatment is implemented by, e.g., crystallization, spray drying, or freeze drying. In one preferred embodiment, the solid antibody formulation is, e.g., in the form of lyophilized powder for injection. The solid antibody formulation can be reconstituted in a suitable vehicle prior to use to give the reconstituted formulation disclosed herein. The reconstituted formulation is also a liquid antibody formulation disclosed herein. In one embodiment, the suitable vehicle is selected from water for injection, organic solvents for injection (including but not limited to, oil for injection, ethanol, propylene glycol, and the like), and a combination thereof.

The liquid formulation disclosed herein can be stably stored for a long period of time, e.g., at least 24 months or longer. In one embodiment, the liquid formulation disclosed herein can be stably stored at about -80 °C to about 45 °C, e.g., -80 °C, about -30 °C, about -20 °C, about 0 °C, about 5 °C, about 25 °C, about 35 °C, about 38 °C, about 40 °C, about 42 °C or about 45 °C for at least 10 days, at least 20 days, at least 1 month, at least 2 months, at least 3 months, at least 4 months, at least 5 months, at least 6 months, at least 7 months, at least 8 months, at least 9 months, at least 10 months, at least 11 months, at least 12 months, at least 18 months, at least 24 months, at least 36 months, or longer.

In one embodiment, the liquid formulation disclosed herein can be stably stored for at least 24 months. In another embodiment, the liquid formulation disclosed herein is stable at a temperature of at least 40 °C. In another embodiment, the liquid formulation disclosed herein remains stable at about 2-8 °C for at least 12 months, preferably at least 24 months. In one embodiment, the liquid formulation disclosed herein remains stable at room temperature or, e.g., about 25 °C for at least 3 months, preferably at least 6 months. In another embodiment, the liquid formulation disclosed herein remains stable at about 40 °C for at least 1 month.

In one embodiment, the stability of the formulation can be indicated after storage by detecting changes in the appearance, visible particles, protein content, purity and/or charge variants of the formulation. In one embodiment, the stability of the liquid formulation disclosed herein can be tested in a high-temperature stress test, e.g., after storage at 40±2 °C for at least 1 week, 2 weeks or preferably 1 month, or after storage at 25±2 °C for at least 1 month or 2 months.

In one embodiment, the stability of the liquid formulation disclosed herein is visually inspected after storage, wherein the liquid formulation disclosed herein remains clear to slightly opalescent in appearance, and it is a colorless to pale yellow liquid and free of particles. In one embodiment, no visible particles exist in the formulation upon visual inspection under a clarity detector. In one embodiment, the stability of the liquid formulation disclosed herein is tested after storage by determining the change in protein content, wherein the change rate in protein content is no more than 20%, preferably no more than 10%, e.g., 7-8%, preferably no more than 5% relative to an initial value on day 0 of storage, as measured, for example, by the ultraviolet spectrophotometry (UV) method. In one embodiment, the stability of the liquid formulation disclosed herein is tested after storage by determining the change in turbidity of the liquid formulation disclosed herein, wherein the change is no more than 0.04, preferably no more than 0.03, and more preferably no more than 0.02, relative to an initial value on day 0 of storage, as measured, for example, by the OD_{350 mm} method. In one embodiment, the stability of the liquid formulation disclosed herein is tested after storage by determining the change in purity of the liquid formulation disclosed herein, wherein the change in monomer purity is no more than 10%, e.g., no more than 5%, 4% or 3%, e.g., 1-2%, preferably no more than 1%, relative to an initial value on day 0 of storage, as measured by size exclusion high performance liquid chromatography (SEC-HPLC). In one embodiment, the stability of the liquid formulation disclosed herein is tested after storage by determining the change in purity of the formulation disclosed herein, wherein the change in monomer purity is reduced by no more than 10%, e.g., no more than 9.5%, 8.5%, 7.5% or 6.5%, as measured by non-reduced and/or reduced capillary electrophoresis-sodium dodecyl sulfate (CE-SDS). In one embodiment, the stability of the liquid formulation disclosed herein is tested after storage by imaged capillary isoelectric focusing (iCIEF), wherein the total change in charge variants (principal component, acidic component and basic component) of the antibody is no more than 50%, e.g., no more than 45%, 40%, 35%, 30% or 25%, relative to an initial value on day 0 of storage. In one embodiment, the stability of the liquid formulation disclosed herein is tested after storage by cation exchange high performance liquid chromatography (CEX-HPLC), wherein the total change in the charge variants (principal component, acidic component and basic component) of the antibody is no more than 40%, e.g., no more than 38%, 36%, 34%, 32% or 30%, relative to an initial value on day 0 of storage.

In one embodiment, the formulation is stable after storage, e.g., at 2-8 °C for at least 24 months, at room temperature for at least 3 months, or at 40±2 °C for 1 month, and preferably, has one or more of the following characteristics:
(i) a purity greater than 90%, preferably greater than 95%, 96%, 97%, 98% or 99%, as measured by SEC-HPLC;
(ii) a purity greater than 85%, preferably greater than 86%, 87%, 88% or 89% as measured by reduced or non-reduced CE-SDS;
(iii) total change ≤ 50% in components (principal component, acidic component and basic component) of the anti-CD47/PD-L1 bispecific antibody protein in the formulation, e.g., ≤ 45%, 40%, 35%, 30% or 25%, relative to an initial value on day 0 of storage, as measured by iCIEF;
(iv) total change ≤ 40% in components (principal component, acidic component and basic component) of the anti-CD47/PD-L1 bispecific antibody protein in the formulation, e.g., ≤ 38%, 36%, 34%, 32% or 30%, relative to an initial value on day 0 of storage, as measured by CEX-HPLC; and
(v) relative binding activity of the anti-CD47/PD-L1 bispecific antibody protein in the formulation of 70-130%, e.g., 70%, 80%, 90%, 100%, 110%, 120% or 130%, relative to an initial value on day 0 of storage, as measured by ELISA.

In one aspect, the present invention provides a delivery device comprising the liquid antibody formulation or the solid antibody formulation disclosed herein. In one embodiment, the delivery device disclosed herein is provided in the form of a pre-filled syringe comprising the liquid antibody formulation or the solid antibody formulation disclosed herein, e.g., for use in intravenous, subcutaneous, intradermal or intramuscular injection, or intravenous infusion.

In another aspect, the present invention provides a method for delivering the anti-CD47/PD-L1 bispecific antibody protein to a subject, e.g., a mammal, which comprises administering the liquid antibody formulation or the solid antibody formulation disclosed herein to the subject, the delivery being implemented, e.g., using a delivery device in the form of a pre-filled syringe.

In another aspect, the present invention provides use of the liquid antibody formulation or the solid antibody formulation disclosed herein in preparing a delivery device (e.g. a pre-filled syringe) or a medicament for treating, preventing or delaying a disorder associated with the SIRPα/CD47 signaling pathway and the PD1/PD-L1 signaling pathway, wherein the disorder includes: various blood diseases and solid tumors, including but not limited to acute myeloid leukemia (AML), chronic myeloid leukemia, acute lymphocytic leukemia (ALL), non-Hodgkin's lymphoma (NHL), multiple myeloma (MM), lymphoma, breast cancer, gastric cancer, lung cancer, esophageal cancer, intestinal cancer, ovarian cancer, cervical cancer, renal cancer, pancreatic cancer, bladder cancer, neuroglioma, melanoma and other solid tumors; autoimmune diseases and inflammatory disorders, e.g., allergic asthma or ulcerative colitis; and rejection for cell or tissue or organ transplant, including rejection for non-human tissue transplant (heterograft).

Other embodiments of the present invention will become apparent by reference to the detailed description hereinafter.

### BRIEF DESCRIPTION OF THE DRAWINGS

The preferred embodiments of the present invention described in detail below will be better understood when read in conjunction with the following drawings. For the purpose of illustrating the present invention, currently preferred embodiments are shown in the drawings. However, it should be understood that the present invention is not limited to accurate arrangement and means of the embodiments shown in the drawings.

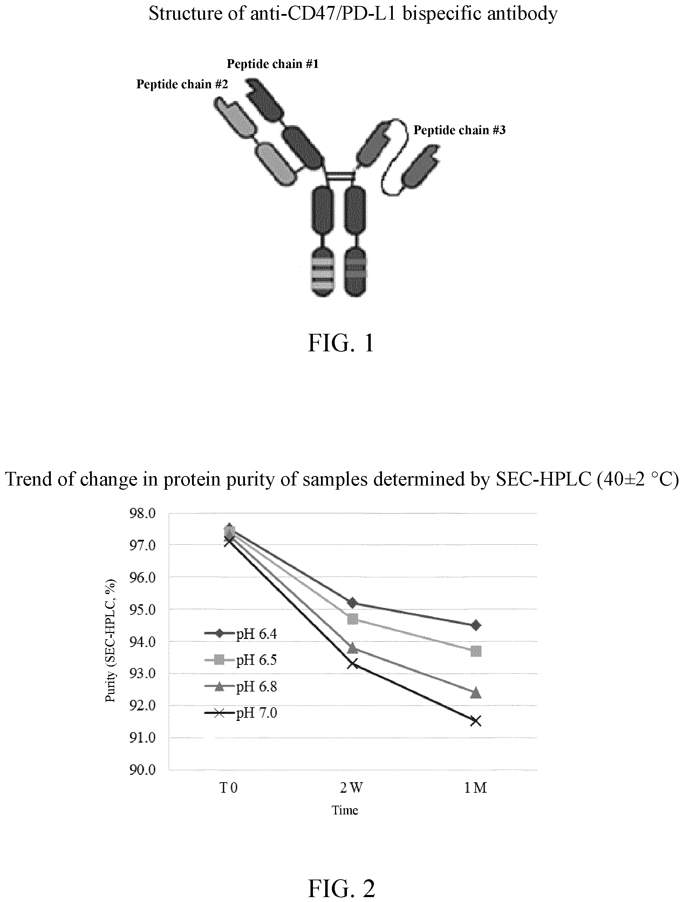

FIG. 1 illustrates the structure of an anti-CD47/PD-L1 bispecific antibody, wherein a first polypeptide chain is paired with a second polypeptide chain to form a first antigen-binding site, and a third polypeptide chain comprises a second single domain antigen-binding site and a third single domain antigen-binding site, and the second single domain antigen-binding site and the third single domain antigen-binding site of the third polypeptide chain have a flexible linker peptide therebetween.
FIG. 2 shows the trend of change in the protein purity of the samples of the anti-CD47/PD-L1 bispecific antibody formulation at pH 6.4, 6.5, 6.8 and 7.0 after storage at 40±2 °C for different time periods, as determined by SEC-HPLC. On the abscissa, T0 represents day 0, 2W represents 2 weeks, and 1M represents 1 month.

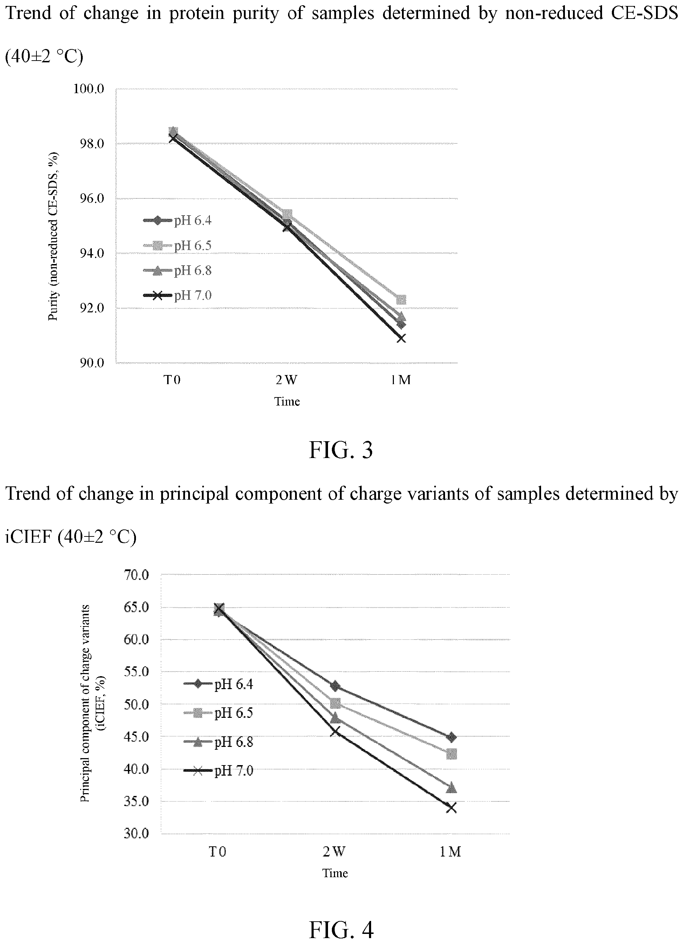

FIG. 3 shows the trend of change in the protein purity of the samples of the anti-CD47/PD-L1 bispecific antibody formulation at pH 6.4, 6.5, 6.8 and 7.0 after storage at 40±2 °C for different time periods, as determined by non-reduced CE-SDS. On the abscissa, T0 represents day 0, 2W represents 2 weeks, and 1M represents 1 month.
FIG. 4 shows the trend of change in the principal component of charge variants of the samples of the anti-CD47/PD-L1 bispecific antibody formulation at pH 6.4, 6.5, 6.8 and 7.0 after storage at 40±2 °C for different time periods, as determined by iCIEF. On the abscissa, T0 represents day 0, 2W represents 2 weeks, and 1M represents 1 month.

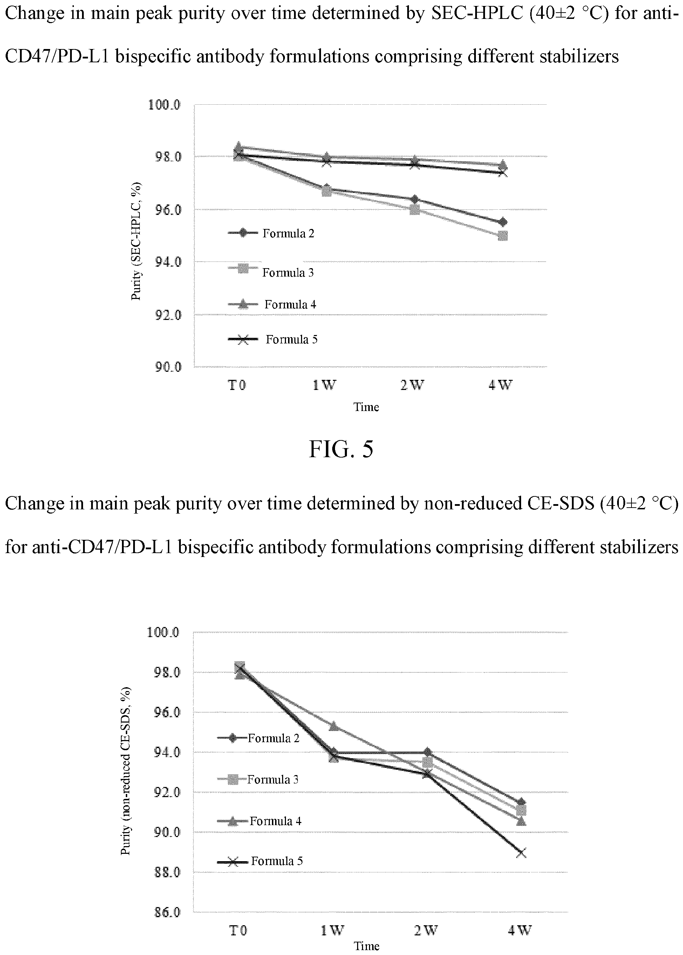

FIG. 5 shows the change in the main peak purity over time of the anti-CD47/PD-L1 bispecific antibody formulations comprising different stabilizers (formulas 2-5) after storage at 40±2 °C for 0 days, 1 week, 2 weeks and 4 weeks, as determined by SEC-HPLC. On the abscissa, T0 represents day 0, 1W represents 1 week, 2W represents 2 weeks, and 4W represents 4 weeks.

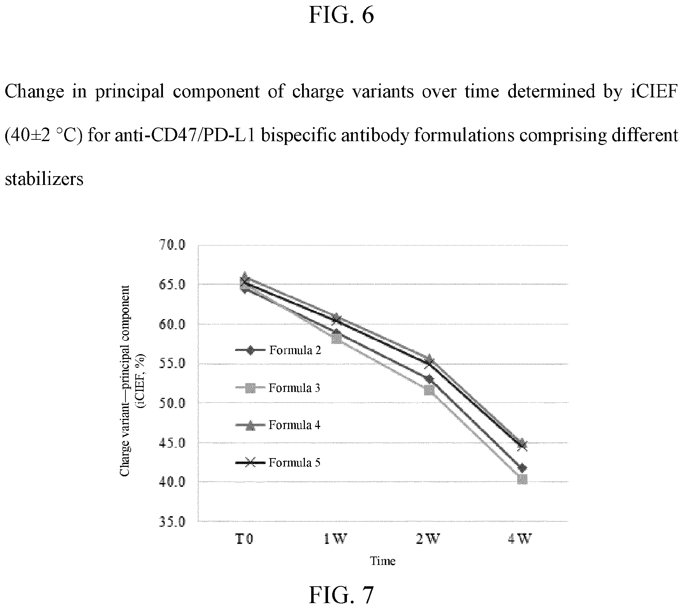

FIG. 6 shows the change in the main peak purity over time of the anti-CD47/PD-L1 bispecific antibody formulations comprising different stabilizers (formulas 2-5) after storage at 40±2 °C for 0 days, 1 week, 2 weeks and 4 weeks, as determined by non-reduced CE-SDS. On the abscissa, T0 represents day 0, 1W represents 1 week, 2W represents 2 weeks, and 4W represents 4 weeks.
FIG. 7 shows the change in the principal component of charge variant over time of the anti-CD47/PD-L1 bispecific antibody formulations comprising different stabilizers (formulas 2-5) after storage at 40±2 °C for 0 days, 1 week, 2 weeks and 4 weeks, as determined by iCIEF. On the abscissa, T0 represents day 0, 1W represents 1 week, 2W represents 2 weeks, and 4W represents 4 weeks.

### DETAILED DESCRIPTION

Before the present invention is described in detail, it should be understood that the present invention is not limited to the particular methodology or experimental conditions described herein, as such methods and conditions may vary. Further, the terms used herein are for the purpose of describing particular embodiments only and are not intended to be limiting.

### Definitions

Unless defined otherwise, all technical and scientific terms used herein have the same meaning as commonly understood by those of ordinary skill in the art. For the purposes of the present invention, the following terms are defined below.

The term "about" used in combination with a numerical value is intended to encompass the numerical values in a range from a lower limit less than the specified numerical value by 5% to an upper limit greater than the specified numerical value by 5%.

The term "and/or", when used to connect two or more options, should be understood to refer to any one of the options or any two or more of the options.

As used herein, the term "comprise" or "comprising" is intended to include the described elements, integers or steps, but not to exclude any other elements, integers or steps. As used herein, the term "comprise" or "comprising", unless indicated otherwise, also encompasses the situation where the entirety consists of the described elements, integers or steps. For example, when referring to an antibody variable region "comprising" a particular sequence, it is also intended to encompass an antibody variable region consisting of the particular sequence.

As used herein, the term "antibody" is used in the broadest sense, and it refers to a protein comprising an antigen-binding site and encompasses natural and artificial antibodies with various structures, including but not limited to triple-chain antibodies, intact antibodies and antigen-binding fragments of antibodies.

The terms "whole antibody", "full-length antibody", "complete antibody" and "intact antibody" are used interchangeably herein to refer to a glycoprotein comprising at least two heavy chains (H) and two light chains (L) interconnected by disulfide bonds. Each heavy chain consists of a heavy chain variable region (abbreviated herein as VH) and a heavy chain constant region. Each heavy chain constant region consists of 3 domains CH1, CH2 and CH3. Each light chain consists of a light chain variable region (abbreviated herein as VL) and a light chain constant region. Each light chain constant region consists of one domain CL. The VH region and the VL region can be further divided into hypervariable regions (complementarity determining regions, or CDRs), with relatively conservative regions (framework regions, or FRs) inserted therebetween. Each VH or VL consists of three CDRs and four FRs, arranged from amino-terminus to carboxyl-terminus in the following order: FR1, CDR1, FR2, CDR2, FR3, CDR3 and FR4. The constant regions are not directly involved in binding of antibodies to antigens, but exhibit a variety of effector functions.

The term "antibody formulation" refers to a preparation which is in a form that allows the biological activity of an antibody as an active ingredient to be exerted effectively, and does not contain other components having unacceptable toxicity to a subject to which the formulation is to be administered. Such antibody formulations are generally sterile. Generally, the antibody formulation comprises a pharmaceutically acceptable excipient. A "pharmaceutically acceptable" excipient is an agent that can be reasonably administered to a mammal subject so that an effective dose of the active ingredient used in the formulation can be delivered to the subject. The concentration of the excipient is adapted to the mode of administration and may, for example, be acceptable for injection.

The term "anti-CD47/PD-L1 bispecific antibody formulation", herein also referred to as the "antibody formulation disclosed herein", refers to a preparation comprising an anti-CD47/PD-L1 bispecific antibody protein as an active ingredient and a pharmaceutically acceptable excipient. The anti-CD47/PD-L1 bispecific antibody protein, as the active ingredient, is suitable for therapeutic or prophylactic administration to a human or non-human animal after the anti-CD47/PD-L1 bispecific antibody protein is combined with the pharmaceutically acceptable excipient. The antibody formulation disclosed herein can be prepared, for example, as an aqueous liquid formulation, e.g., in a ready-to-use pre-filled syringe, or as a lyophilized formulation to be reconstituted (i.e., redissolved) by dissolution and/or suspension in a physiologically acceptable solution immediately prior to use. In some embodiments, the anti-CD47/PD-L1 bispecific antibody protein formulation is in the form of a liquid formulation.

A "stable" antibody formulation is a formulation where the antibody retains an acceptable degree of physical and/or chemical stability after storage under specific conditions. Although the antibody in the antibody formulation may not maintain 100% of its chemical structure after storage for a specific period of time, the antibody formulation is considered "stable" when the antibody typically maintains about 90%, about 95%, about 96%, about 97%, about 98%, or about 99% of its structure or function after storage for a specific period of time. In some specific embodiments, the antibody aggregation or degradation or chemical modification is barely detected in the anti-CD47/PD-L1 bispecific antibody protein formulation disclosed herein during manufacture, formulation, transportation and long-term storage, resulting in little or even no loss of biological activity of the anti-CD47/PD-L1 bispecific antibody protein and exhibiting high stability. In some embodiments, the anti-CD47/PD-L1 bispecific antibody protein formulation disclosed herein substantially retains its physical and chemical stability after storage. Preferably, the liquid formulation disclosed herein can remain stable at room temperature or at 40 °C for at least 1 month and/or at 2-8 °C for at least 24 months.

A variety of analytical techniques are known in the art for determining the stability of proteins, see, e.g., Peptide and Protein Drug Delivery, 247-301, Vincent Lee Ed., Marcel Dekker, Inc., New York, N.Y., Pubs (1991) and Jones, A. Adv. Drug Delivery Rev. 10: 29-90 (1993). Stability can be determined at a selected temperature and for a selected storage time. For example, the storage time can be selected based on the expected shelf life of the formulation. Alternatively, an accelerated stability test can be adopted. In some embodiments, the stability test is performed by conducting various stress tests on the antibody formulation. These tests can represent extreme conditions that a formulated antibody formulation may be subjected to during manufacture, storage or transportation, and can also represent conditions that may accelerate the instability of the antibody in the antibody formulation during non-manufacture, storage or transportation. For example, the formulated anti-CD47/PD-L1 bispecific antibody protein formulation can be filled into a glass vial to test the stability of the antibody under high temperature stress.

The antibody can be considered to "maintain its physical stability" in the formulation if the formulation does not exhibit aggregation, precipitation, turbidity and/or denaturation, or exhibits very little aggregation, precipitation, turbidity, and/or denaturation after storage for a period of time. Safety issues arise as the aggregation of antibodies in the formulation can potentially lead to an increased immune response in a patient. Accordingly, there is a need to minimize or prevent the aggregation of antibodies in the formulation. Light scattering methods can be used to determine visible aggregates in the formulation. SEC can be used to determine soluble aggregates in the formulation. In addition, the stability of the formulation can be indicated by visually inspecting the appearance, color and/or clarity of the formulation, or by detecting the turbidity of the formulation by the OD_{350 nm} method, or by determining the purity of the formulation by non-reduced CE-SDS. In one embodiment, the stability of the formulation is measured by determining the percentage of antibody monomer in the formulation after storage at a particular temperature for a particular period of time, wherein the higher the percentage of antibody monomer in the formulation, the higher the stability of the formulation.

An "acceptable degree" of physical stability can represent that at least about 92% of anti-CD47/PD-L1 bispecific antibody protein monomer is detected in the formulation after storage at a specific temperature for a specific period of time. In some embodiments, an acceptable degree of physical stability represents at least about 88%, 89%, 90%, 91%, 92%, 93%, 94%, 95%, 96%, 97%, 98% or 99% of anti-CD47/PD-L1 bispecific antibody protein monomer after storage at a specific temperature for at least 2 weeks, at least 28 days, at least 1 month, at least 2 months, at least 3 months, at least 4 months, at least 5 months, at least 6 months, at least 7 months, at least 8 months, at least 9 months, at least 10 months, at least 11 months, at least 12 months, at least 18 months, at least 24 months, or longer. When physical stability is assessed, the specific temperature at which the pharmaceutical formulation is stored can be any temperature from about -80 °C to about 45 °C, e.g., at about -80 °C, about -30 °C, about -20 °C, about 0 °C, about 4-8 °C, about 5 °C, about 25 °C, about 35 °C, about 37 °C, about 40 °C, about 42 °C, or about 45 °C. For example, a pharmaceutical formulation is considered stable if at least about 88%, 89%, 90%, 91%, 92%, 93%, 94%, 95%, 96%, 97%, 98% or 99% of anti-CD47/PD-L1 bispecific antibody protein monomer is detected after storage at about 40±2 °C for 1 month or 4 weeks. A pharmaceutical formulation is considered stable if at least about 88%, 89%, 90%, 91%, 92%, 93%, 94%, 95%, 96%, 97%, 98% or 99% of anti-CD47/PD-L1 bispecific antibody protein monomer is detected after storage at about 25 °C for 2 months. A pharmaceutical formulation is considered stable if at least about 88%, 89%, 90%, 91%, 92%, 93%, 94%, 95%, 96%, 97%, 98% or 99% of anti-CD47/PD-L1 bispecific antibody protein monomer is detected after storage at about 5 °C for 9 months.

The antibody can be considered to "maintain its chemical stability" in the formulation if the antibody in the formulation does not exhibit significant chemical changes after storage for a period of time. Most of the chemical instability results from the formation of covalently modified forms of the antibody (e.g., charge variants of the antibody). Basic variants can be formed, for example, by aspartic acid isomerization, and N- and C-terminal modifications; acidic variants can be produced by deamidation, sialylation and glycation. Chemical stability can be assessed by detecting and/or quantifying chemically altered forms of the antibody. For example, charge variants of the antibody in the formulation can be detected by cation exchange chromatography (CEX) or imaged capillary isoelectric focusing (iCIEF). In one embodiment, the stability of the formulation is measured by determining the percentage change in charge variants of the antibody in the formulation after storage at a specific temperature for a specific period of time, wherein the smaller the change, the higher the stability of the formulation.

An "acceptable degree" of chemical stability can represent the percentage change in charge variants (e.g., principal component, acidic component or basic component) in the formulation of no more than 30%, e.g., 20%, after storage at a specific temperature for a specific period of time. In some embodiments, an acceptable degree of chemical stability can represent the percentage change in charge variant (acidic component) of no more than about 25%, 20%, 15%, 10%, 5%, 4%, 3%, 2% or 1% after storage at a specific temperature for at least 2 weeks, at least 28 days, at least 1 month, at least 2 months, at least 3 months, at least 4 months, at least 5 months, at least 6 months, at least 7 months, at least 8 months, at least 9 months, at least 10 months, at least 11 months, at least 12 months, at least 18 months, at least 24 months, or longer. When chemical stability is assessed, the temperature at which the pharmaceutical formulation is stored can be any temperature from about -80 °C to about 45 °C, e.g., at about -80 °C, about -30 °C, about -20 °C, about 0 °C, about 4-8 °C, about 5 °C, about 25 °C, or about 45 °C. For example, the pharmaceutical formulation can be considered stable if the percentage change in charge variant acidic component is less than about 25%, 24%, 23%, 22%, 21%, 20%, 19%, 18%, 17%, 16%, 15%, 14%, 13%, 12%, 10%, 9%, 8%, 7%, 6%, 5%, 4%, 3%, 2%, 1%, 0.5% or 0.1% after storage at 5 °C for 24 months. The pharmaceutical formulation can also be considered stable if the percentage change in charge variant acidic component is less than about 20%, 19%, 18%, 17%, 16%, 15%, 14%, 13%, 12%, 10%, 9%, 8%, 7%, 6%, 5%, 4%, 3%, 2%, 1%, 0.5% or 0.1% after storage at 25 °C for 2 months. The pharmaceutical formulation can also be considered stable if the percentage change in charge variant acidic component is less than about 25%, 24%, 23%, 22%, 21%, 20%, 19%, 18%, 17%, 16%, 15%, 14%, 13%, 12%, 10%, 9%, 8%, 7%, 6%, 5%, 4%, 3%, 2%, 1%, 0.5% or 0.1% after storage at 40 °C for 1 month.

The term "lyophilized formulation" refers to a composition obtained or obtainable by a freeze-drying process of a liquid formulation. Preferably, it is a solid composition having a water content of less than 5%, preferably less than 3%.

The term "reconstituted formulation" refers to a liquid formulation obtained by dissolving and/or suspending a solid formulation (e.g., a lyophilized formulation) in a physiologically acceptable solution.

As used herein, the term "room temperature" refers to a temperature from 15 °C to 30 °C, preferably from 20 °C to 27 °C, more preferably 25 °C.

"Stress conditions" refer to environments that are chemically and/or physically unfavorable to antibody proteins and may result in unacceptable destabilization of the antibody proteins. "High temperature stress" refers to storing the antibody formulation at room temperature or higher (e.g., 40±2 °C) for a period of time. The stability of the antibody formulation can be tested by the high-temperature stress accelerated test.

As used herein, the term "parenteral administration" refers to administrations other than enteral and topical administrations, typically by injection or infusion, including but not limited to, intravenous, intramuscular, intraarterial, intrathecal, intracapsular, intraorbital, intracardiac, intradermal, intraperitoneal, transtracheal, subcutaneous, subcuticular, intraarticular, subcapsular, subarachnoid, intraspinal, epidural and intrasternal injection and infusion. In some embodiments, the stable anti-CD47/PD-L1 bispecific antibody protein formulation disclosed herein is administered parenterally to a subject. In one embodiment, the anti-CD47/PD-L1 bispecific antibody protein formulation disclosed herein is administered by subcutaneous, intradermal, intramuscular or intravenous injection to a subject.

### I. Antibody Formulation

The present invention provides a stable liquid antibody formulation comprising (i) an anti-CD47/PD-L1 bispecific antibody protein, (ii) a buffer, (iii) a stabilizer, (iv) a surfactant, and optionally (v) other excipients, wherein the pH of the antibody formulation is about 6.4-7.0. In one preferred embodiment, the liquid antibody formulation disclosed herein is in the form of an injection.

### (i) Anti-CD47/PD-L1 bispecific antibody protein

The anti-CD47/PD-L1 bispecific antibody protein in the antibody formulation disclosed herein is a triple-chain antibody, which comprises a VH/VL pair specifically binding to CD47 on a first polypeptide chain and a second polypeptide chain as a first antigen-binding site, and a first VHH and a second VHH specifically binding to PD-L1 on a third polypeptide chain as a second single domain antigen-binding site and a third single domain antigen-binding site, respectively; or comprises a VH/VL pair specifically binding to PD-L1 on a first polypeptide chain and a second polypeptide chain as a first antigen-binding site, and a first VHH and a second VHH specifically binding to CD47 on a third polypeptide chain as a second single domain antigen-binding site and a third single domain antigen-binding site, respectively. The anti-CD47/PD-L1 bispecific antibody protein is capable of binding to CD47 with an affinity constant of at least about 10 M⁻¹, preferably about 10⁸ M⁻¹, and more preferably about 10⁹ M⁻¹ or greater, and is capable of binding to PD-L1 with an affinity constant of at least about 10⁷ M⁻¹, preferably about 10 M⁻¹, and more preferably about 10⁹ M⁻¹ or greater, such that the antibody can be used as a therapeutic agent and/or a prophylactic agent featuring bispecific targeting of CD47 and PD-L1 molecules.

The VH/VL pair specifically binding to PD-L1 or CD47 comprises 6 CDRs of a VH/VL pair of an anti-PD-L1 antibody reported in any prior art and an anti-PD-L1 antibody developed in the future, or sequences having one, two, three, four, five, six or more amino acid changes (e.g., amino acid substitutions or deletions) compared with one or more CDRs of the 6 CDRs; or comprises 6 CDRs of a VH/VL pair of an anti-CD47 antibody reported in any prior art and an anti-CD47 antibody developed in the future, or sequences having one, two, three, four, five, six or more amino acid changes (e.g., amino acid substitutions or deletions) compared with one or more CDRs of the 6 CDRs.

The first VHH and the second VHH that specifically bind to PD-L1 or CD47 are both derived from a heavy chain variable domain of an antibody that naturally devoid of light chains (e.g., a heavy chain variable domain of a heavy chain antibody naturally occurring in the Camelidae species). The first VHH and the second VHH may be the same or different. The first VHH and the second VHH may be derived from antibodies produced in Camelidae species, such as camels, alpacas, dromedaries, llamas and guanacos. Species other than Camelidae may also produce heavy chain antibodies naturally devoid of light chains, and such VHHs are also within the scope of the antibody protein disclosed herein. The first VHH and the second VHH comprise 3 CDRs of a VHH of an anti-PD-L1 antibody reported in any prior art and an anti-PD-L1 antibody developed in the future, or sequences having one, two, three, four, five, six or more amino acid changes (e.g., amino acid substitutions or deletions) compared with one or more CDRs of the 3 CDRs; or comprise 3 CDRs of a VHH of an anti-CD47 antibody reported in any prior art and an anti-CD47 antibody developed in the future, or sequences having one, two, three, four, five, six or more amino acid changes (e.g., amino acid substitutions or deletions) compared with one or more CDRs of the 3 CDRs.

In one embodiment, the VH/VL pair specifically binding to CD47 on the first polypeptide chain and the second polypeptide chain of the anti-CD47/PD-L1 bispecific antibody protein comprises a VH CDR1 set forth in GSIEHYYWS (SEQ ID NO: 3), a VH CDR2 set forth in YIYYSGSTNYNPSLKS (SEQ ID NO: 4), a VH CDR3 set forth in ARGKTGSAA (SEQ ID NO: 5), a VL CDR1 set forth in RASQGISRWLA (SEQ ID NO: 10), a VL CDR2 set forth in AASSLQS (SEQ ID NO: 11) and a VL CDR3 set forth in QQTVSFPIT (SEQ ID NO: 12) derived from the anti-CD47 antibody ADI-29341 reported in Chinese Patent Application No. CN201710759828.9, or sequences having one, two, three, four, five, six or more amino acid changes (e.g., amino acid substitutions or deletions) compared with one or more CDRs of the 6 CDRs.

In one embodiment, the first VHH and the second VHH specifically binding to PD-L1 on the third polypeptide chain of the anti-CD47/PD-L1 bispecific antibody protein both comprise a CDR1 set forth in AYTISRNSMG (SEQ ID NO: 17), a CDR2 set forth in IESDGST (SEQ ID NO: 18) and a CDR3 set forth in AAPKVGLGPRTALGHLAFMTLPALNY (SEQ ID NO: 19), or sequences having one, two, three, four, five, six or more amino acid changes (e.g., amino acid substitutions or deletions) compared with one or more CDRs of the 3 CDRs.

The term "CDR", "complementarity determining region" or "CDR region" (used interchangeably herein with a hypervariable region "HVR") refers to an amino acid region in the variable region of an antibody that is primarily responsible for binding to an epitope of an antigen. The CDRs of the heavy and light chains are generally referred to as CDR1, CDR2, and CDR3, and are numbered sequentially from the N-terminus. Various schemes for determining the CDR sequence of a given VH, VL or VHH amino acid sequence are known in the art. For example, Kabat complementarity determining regions (CDRs) are determined based on sequence variability and are the most commonly used (Kabat et al., Sequences of Proteins of Immunological Interest, 5th Ed. Public Health Service, National Institutes of Health, Bethesda, Md. (1991)). Chothia scheme is based on the positions of structural loops (Chothia and Lesk, J. mol. biol. 196:901-917 (1987)). AbM HVRs are a compromise between Kabat HVRs and Chothia structural loops and are used by Oxford Molecular's AbM antibody modeling software. The "contact" HVRs are based on analysis of available complex crystal structures. HVRs can also be determined based on the same Kabat numbering position as the reference CDR sequences (e.g., exemplary CDRs disclosed herein).

The amino acid changes, e.g., amino acid substitutions, are preferably conservative amino acid replacements. The "conservative amino acid replacement" refers to an amino acid alteration that results in the substitution of an amino acid with a chemically similar amino acid. Conservative substitution tables providing functionally similar amino acids are well known in the art. In any of the embodiments herein, in one preferred aspect, the conservatively replaced residue is from the conservative replacement Table A below, preferably the preferred substituted residues shown in Table A.

**Table A**

| Original residues | Exemplary replacement | Preferred conservative amino acid replacement |
|---|---|---|
| Ala (A) | Val; Leu; Ile | Val |
| Arg (R) | Lys; Gln; Asn | Lys |
| Asn (N) | Gln; His; Asp; Lys; Arg | Gln |
| Asp (D) | Glu; Asn | Glu |
| Cys (C) | Ser; Ala | Ser |
| Gln (Q) | Asn; Glu | Asn |
| Glu (E) | Asp; Gln | Asp |
| Gly (G) | Ala | Ala |
| His (H) | Asn; Gln; Lys; Arg | Arg |
| Ile (I) | Leu; Val; Met; Ala; Phe; Nle | Leu |
| Leu (L) | Nle; Ile; Val; Met; Ala; Phe | Ile |
| Lys (K) | Arg; Gln; Asn | Arg |
| Met (M) | Leu; Phe; Ile | Leu |
| Phe (F) | Trp; Leu; Val; Ile; Ala; Tyr | Tyr |
| Pro (P) | Ala | Ala |
| Ser (S) | Thr | Thr |
| Thr (T) | Val; Ser | Ser |
| Trp (W) | Tyr; Phe | Tyr |
| Tyr (Y) | Trp; Phe; Thr; Ser | Phe |
| Val (V) | Ile; Leu; Met; Phe; Ala; Nle | Leu |

In one embodiment, the VH/VL pair specifically binding to CD47 on the first polypeptide chain and the second polypeptide chain of the anti-CD47/PD-L1 bispecific antibody protein comprises paired heavy chain variable region/light chain variable region sequences of SEQ ID NOs: 2/9 derived from anti-CD47 antibody ADI-29341, or sequences having at least 90%, 91%, 92%, 93%, 94%, 95%, 96%, 97%, 98%, 99% or higher sequence identity to the paired heavy chain variable region/light chain variable region sequences.

In one embodiment, the first VHH and the second VHH specifically binding to PD-L1 on the third polypeptide chain of the anti-CD47/PD-L1 bispecific antibody protein comprise amino acid sequences set forth in SEQ ID NO: 15 and/or SEQ ID NO: 16, or sequences substantially identical (e.g., having at least 80%, 85%, 90%, 92%, 95%, 97%, 98%, 99% or higher identity) thereto.

The type of the heavy chain constant region of the immunoglobulin in the first polypeptide chain and the third polypeptide chain in the anti-CD47/PD-L1 bispecific antibody protein disclosed herein is not particularly limited, and is preferably a heavy chain constant region of the IgG1, IgG2 or IgG4 immunoglobulin, or a sequence substantially identical (e.g., having at least 80%, 85%, 90%, 92%, 95%, 97%, 98%, 99% or higher identity) thereto. More preferably, the heavy chain constant region is a heavy chain constant region of a human IgG1 immunoglobulin, or a sequence substantially identical (for example, at least 80%, 85%, 90%, 92%, 95%, 97%, 98%, 99%, or higher identity) thereto.

In one embodiment, the anti-CD47/PD-L1 bispecific antibody protein comprises a heavy chain constant region used in IgG4 (e.g., human IgG4). In yet another embodiment, the anti-CD47/PD-L1 bispecific antibody protein comprises a heavy chain constant region used in IgG1 (e.g., human IgG1). For example, each Fc domain of the first polypeptide chain and the third polypeptide chain of the triple-chain antibody comprises a hinge region having a "CPPC" amino acid sequence, and/or each comprises Y349C and S354C (according to the "EU numbering" of Kabat), whereby the first polypeptide chain and the third polypeptide chain form interchain disulfide bonds in the Fc region and thus stabilize the correct pairing of the first polypeptide chain and the third polypeptide chain.

In one embodiment, the first polypeptide chain and/or the third polypeptide chain of the anti-CD47/PD-L1 bispecific antibody protein comprise amino acid mutations in the Fc domain which affect the function of the antibody effector. In one specific embodiment, the effector function is antibody-dependent cell-mediated cytotoxicity (ADCC). In one embodiment, the amino acid mutation is present in the CH2 domain of the Fc region. For example, the anti-CD47/PD-L1 bispecific antibody protein comprises amino acid substitutions at positions 234 and 235 (EU numbering) of the first polypeptide chain and/or the third polypeptide chain. In one specific embodiment, the amino acid substitutions are L234A and L235A (also referred to as "LALA mutations").

In yet another embodiment, the second polypeptide chain of the anti-CD47/PD-L1 bispecific antibody protein comprises a kappa light chain constant region or a lambda light chain constant region, for example, a human kappa light chain constant region or a human lambda light chain constant region.

In one embodiment, the first polypeptide chain and the third polypeptide chain of the anti-CD47/PD-L1 bispecific antibody protein comprise a protuberance ("knob") and a cavity ("hole") respectively in their Fc domains, or vice versa, and the protuberance (or cavity) in the Fc domain of the first polypeptide chain can be placed in the cavity (or protuberance) in the Fc domain of the third polypeptide chain, such that the first polypeptide chain and the third polypeptide chain form a stable "knob-in-hole" association with each other. In one embodiment, the amino acid substitution T366W is contained in one of the first polypeptide chain and the third polypeptide chain, and the amino acid substitutions T366S, L368A, and Y407V (EU numbering) are contained in the other one of the first polypeptide chain and the third polypeptide chain. Thus, the protuberance in one chain can be placed in the cavity in the other chain, which promotes the correct pairing of the first polypeptide chain and the third polypeptide chain.

In one embodiment, the immunoglobulin CH1 domain and CL domain of the first polypeptide chain and the second polypeptide chain of the anti-CD47/PD-L1 bispecific antibody protein comprise a protuberance and a cavity respectively, or vice versa, and the protuberance (or cavity) in the CH1 domain can be placed in the cavity (or protuberance) in the CL domain, such that the first polypeptide chain and the second polypeptide chain also form a stable "knob-in-hole" association with each other.

In one embodiment, the anti-CD47/PD-L1 bispecific antibody protein comprises a first polypeptide chain set forth in SEQ ID NO: 1, a second polypeptide chain set forth in SEQ ID NO: 8, and a third polypeptide chain set forth in SEQ ID NO: 14, or sequences substantially identical (e.g., having at least 80%, 85%, 90%, 92%, 95%, 97%, 98%, 99% or higher identity) to any one of the sequences.

In yet another embodiment, the anti-CD47/PD-L1 bispecific antibody protein comprises a first polypeptide chain set forth in SEQ ID NO: 1, a second polypeptide chain set forth in SEQ ID NO: 8, and a third polypeptide chain set forth in SEQ ID NO: 22, or sequences substantially identical (e.g., having at least 80%, 85%, 90%, 92%, 95%, 97%, 98%, 99% or higher identity) to any one of the sequences.

As used herein, the term "sequence identity" refers to the degree to which sequences are identical on a nucleotide-by-nucleotide or amino acid-by-amino acid basis in a comparison window. The "percent sequence identity" can be calculated by the following steps: comparing two optimally aligned sequences in a comparison window; determining a number of positions in which nucleic acid bases (e.g., A, T, C, G and I) or amino acid residues (e.g., Ala, Pro, Ser, Thr, Gly, Val, Leu, Ile, Phe, Tyr, Trp, Lys, Arg, His, Asp, Glu, Asn, Gln, Cys, and Met) are the same in the two sequences to give the number of matched positions; dividing the number of matched positions by the total number of positions in the comparison window (i.e., the window size); and multiplying the result by 100 to give a percent sequence identity. Optimal alignment for determining the percent sequence identity can be achieved in a variety of ways known in the art, for example, using publicly available computer software such as BLAST, BLAST-2, ALIGN, or Megalign (DNASTAR) software. Those skilled in the art can determine suitable parameters for alignment of the sequences, including any algorithms necessary to achieve optimal alignment in a full-length sequence range or target sequence region being compared.

The anti-CD47/PD-L1 bispecific antibody protein in the antibody formulation disclosed herein is capable of simultaneously binding to PD-L1 and CD47 proteins and maintains the affinity constant of each parent antibody, so that the SIRPα/CD47 signaling pathway and the PD1/PD-L1 signaling pathway can be blocked, and thus the antibody formulation can be used to treat, prevent or delay various diseases or disorders associated with the SIRPα/CD47 signaling pathway and/or the PD1/PD-L1 signaling pathway.

In one preferred embodiment, the anti-CD47/PD-L1 bispecific antibody protein disclosed herein is a recombinant anti-CD47/PD-L1 bispecific antibody protein disclosed in PCT application No. PCT/CN2018/123886 (filed on Dec. 26, 2018), and it has a first polypeptide chain set forth in SEQ ID NO: 1, a second polypeptide chain set forth in SEQ ID NO: 8, and a third polypeptide chain set forth in SEQ ID NO: 14. In one embodiment, the anti-CD47/PD-L1 bispecific antibody protein is produced by recombinant expression in HEK293 cells or CHO cells and purified. Preferably, the antibody in the liquid formulation disclosed herein exhibits significant anti-tumor activity. The anti-CD47/PD-L1 bispecific antibody is administered to tumor-bearing mice obtained by inoculating NOD-SCID mice with Raji-PD-L1 cells, and results show that compared with the administration of an anti-CD47 monoclonal antibody and an anti-PD-L1 monoclonal antibody, the anti-CD47/PD-L1 bispecific antibody has significantly improved anti-tumor activity, and it can result in a tumor growth inhibition of about 90% or more, e.g., 100%, and/or a tumor disappearance rate of 50% or more. Furthermore, the anti-CD47/PD-L1 bispecific antibody also shows significantly reduced hemagglutination and thus will have significantly reduced side effects in clinical treatment.

The amount of the anti-CD47/PD-L1 bispecific antibody protein in the antibody formulation disclosed herein can vary with the specific desired characteristics of the formulation, the specific environment and the specific purpose for which the formulation is used. In some embodiments, the antibody formulation is a liquid formulation, which may comprises about 5-150 mg/mL, e.g., about 5, 10, 20, 30, 40, 50, 60, 70, 80, 90, 100, 110, 120, 130, 140 or 150 mg/mL anti-CD47/PD-L1 bispecific antibody protein.

### (ii) Buffer

Buffers are reagents that can control the pH of a solution within an acceptable range. In some embodiments, the buffer in the formulation disclosed herein can control the pH of the formulation disclosed herein at about 6.4-7.0, e.g., about 6.5. In some specific embodiments, the pH of the antibody formulation disclosed herein is about 6.4, 6.5, 6.6, 6.7, 6.8, 6.9 or 7.0.

In some embodiments, the buffer in the formulation disclosed herein is selected from histidine, histidine hydrochloride and a combination thereof. In one embodiment, the concentration of the buffer in the liquid antibody formulation disclosed herein is about 1-30 mM. In one embodiment, the concentration of the buffer in the liquid antibody formulation disclosed herein is about 5-25 mM, e.g., about 5, 10, 15, 20 or 25 mM.

In one embodiment, the buffer in the formulation disclosed herein is a combination of about 16.3 mM histidine and about 3.77 mM histidine hydrochloride.

### (iii) Stabilizer

Suitable stabilizers for use in the present invention can be selected from saccharides, polyols and amino acids and combinations thereof. Saccharides used as stabilizers include, but are not limited to, sucrose and trehalose. Polyols used as stabilizers include, but are not limited to, sorbitol. Amino acids used as stabilizers include, but are not limited to, arginine and arginine hydrochloride. In some embodiments, the stabilizer is present in the liquid formulation disclosed herein in an amount of about 50-500 mM, preferably about 100-400 mM, e.g., about 100, 150, 200, 250, 300, 350 or 400 mM.

In one embodiment, the liquid formulation disclosed herein comprises sucrose as the stabilizer. The amount of sucrose in the liquid formulation disclosed herein can be about 50-250 mM, preferably about 100-200 mM (e.g., about 100, 110, 120, 130, 140, 150, 160, 170, 180, 190 or 200 mM).

In one embodiment, the liquid formulation disclosed herein comprises arginine and/or arginine hydrochloride as the stabilizer. The amount of arginine and/or arginine hydrochloride in the liquid formulation disclosed herein can be about 50-250 mM, preferably about 100-200 mM (e.g., about 100, 110, 120, 130, 140, 150, 160, 170, 180, 190 or 200 mM).

In one embodiment, the liquid formulation disclosed herein comprises a combination of sucrose, arginine and/or arginine hydrochloride as the stabilizer. In this combination, sucrose may be present in an amount of about 50-250 mM, preferably about 100-200 mM (e.g., about 100, 110, 120, 130, 140, 150, 160, 170, 180, 190 or 200 mM). In this combination, arginine and/or arginine hydrochloride may be present in an amount of about 50-250 mM, preferably about 100-200 mM (e.g., about 100, 110, 120, 130, 140, 150, 160, 170, 180, 190 or 200 mM).

### (iv) Surfactant

As used herein, the term "surfactant" refers to an organic substance with an amphiphilic structure; that is, the structure is composed of groups with opposite solubility tendencies, typically an oil-soluble hydrocarbon chain and a water-soluble ionic group.

In one embodiment, the surfactant in the liquid formulation disclosed herein is a non-ionic surfactant, e.g., alkyl poly(ethylene oxide). Specific non-ionic surfactants that can be included in the formulation disclosed herein include, for example, polysorbates such as polysorbate-20, polysorbate-80, polysorbate-60 or polysorbate-40, Plonik, and the like. In one preferred embodiment, the liquid formulation disclosed herein comprises polysorbate-80 as the surfactant.

The amount of surfactant in the antibody formulation disclosed herein can vary with the specific desired characteristics of the formulation, the specific environment, and the specific purpose for which the formulation is used. In some preferred embodiments, the formulation can comprise about 0.1-1 mg/mL, preferably about 0.2-0.8 mg/mL, e.g., about 0.2, 0.3, 0.4, 0.5, 0.6, 0.7 or 0.8 mg/mL, polysorbate surfactant (e.g., polysorbate-80).

### (v) Other excipients

The antibody liquid formulation disclosed herein may or may not comprise other excipients.

In one embodiment, the antibody liquid formulation disclosed herein comprises a metal chelating agent (e.g., EDTA or a salt thereof) as an excipient. In another embodiment, the antibody liquid formulation disclosed herein does not comprise a metal chelating agent (e.g., EDTA or a salt thereof). In one embodiment, the antibody liquid formulation disclosed herein with a metal chelating agent (e.g., EDTA or a salt thereof) is more stable than a formulation without a metal chelating agent (e.g., EDTA or a salt thereof).

For other considerations, other excipients can also be used in the formulation disclosed herein. Such excipients include, for example, flavoring agents, antimicrobial agents, sweeteners, antistatic agents, antioxidants, gelatin, and the like. These and other known pharmaceutical excipients and/or additives suitable for use in the formulation disclosed herein are well known in the art, for example, as listed in *"*The Handbook of Pharmaceutical Excipients, 4th edition, edited by Rowe et al, American Pharmaceuticals Association (2003); and Remington: the Science and Practice of Pharmacy, 21st edition, edited by Gennaro, Lippincott Williams & Wilkins (2005)".

### II. Preparation of Formulation

The present invention provides a stable formulation comprising an anti-CD47/PD-L1 bispecific antibody protein. The anti-CD47/PD-L1 bispecific antibody protein used in the formulation disclosed herein can be prepared using techniques known in the art for the production of antibodies. For example, the anti-CD47/PD-L1 bispecific antibody protein can be recombinantly prepared. In one preferred embodiment, the anti-CD47/PD-L1 bispecific antibody protein disclosed herein is prepared by recombinant expression in HEK293 cells or CHO cells. For example, the anti-CD47/PD-L1 bispecific antibody protein is recombinantly prepared as described in PCT/CN2018/123886.

The use of antibodies as active ingredients in drugs is now very common. Techniques for purifying therapeutic antibodies to pharmaceutical grade are well known in the art. For example, Tugcu et al. (Maximizing productivity of chromatography steps for purification of monoclonal antibodies, Biotechnology and Bioengineering 99 (2008) 599-613) describes an antibody three-column purification method in which ion exchange chromatography (anionic IEX and/or cationic CEX chromatography) is used after a protein A capture step. Kelley et al (Weak partitioning chromatography for anion exchange purification of monoclonal antibodies, Biotechnology and Bioengineering 101 (2008) 553-566) describes a two-column purification method in which a weak partitioning anion exchange resin is used after protein A affinity chromatography.

Generally, antibodies recombinantly produced can be purified using conventional purification methods to provide a drug substance with sufficient reproducibility and moderate purity for formulating antibody formulations. For example, after the antibody is secreted from the recombinant expression cells into the culture medium, the supernatant from the expression system can be concentrated using a commercially available protein concentration filter, e.g., Amicon ultrafiltration device. Then the antibody can be purified by methods such as chromatography, dialysis, and affinity purification. Protein A is suitable as an affinity ligand for the purification of IgG1, IgG2 and IgG4 antibodies. Other antibody purification methods, such as ion exchange chromatography, can also be used. After the antibody with sufficient purity is obtained, a formulation comprising the antibody can be prepared according to methods known in the art.

For example, the preparation can be performed by the following steps: (1) removing impurities such as cells from fermentation broth by centrifuging and clarifying after the fermentation to obtain a supernatant; (2) capturing an antibody using affinity chromatography (e.g., a protein A column with specific affinity for IgG1, IgG2 and IgG4 antibodies); (3) inactivating viruses; (4) purifying (usually CEX cation exchange chromatography can be adopted) to remove impurities in a protein; (5) filtering the viruses (to reduce the virus titer by, e.g., more than 4 log10); and (6) ultrafiltering/diafiltering (which can be used to allow the protein to be exchanged into a formulation buffer that is favorable for its stability and concentrated to a suitable concentration for injection). See, e.g., B. Minow, P. Rogge, K. Thompson, BioProcess International, Vol. 10, No. 6, 2012, pp. 48-57.

### III. Analytical Method of Formulation

During the storage of antibody formulations, antibodies may undergo aggregation, degradation or chemically modification that results in antibody heterogeneity (including size heterogeneity and charge heterogeneity), aggregates and fragments, etc., and thus affects the quality of the antibody formulations. Accordingly, it is necessary to monitor the stability of antibody formulations.

Various methods are known in the art for testing the stability of antibody formulations. For example, the purity of the antibody formulation can be analyzed and the aggregation level of the antibody can be evaluated by methods such as reduced CE-SDS, non-reduced CE-SDS and SEC-HPLC; charge variants in the antibody formulation can be analyzed by capillary isoelectric focusing electrophoresis (cIEF), imaged capillary isoelectric focusing (iCIEF), ion exchange chromatography (IEX), and the like. In addition, the stability of the formulation can be determined quickly by visually inspecting the appearance of the formulation. The change in turbidity of the formulation can also be detected by the OD_{350 nm} method, which gives information about the amount of soluble and insoluble aggregates. In addition, the change in protein content in the formulation can be detected by the ultraviolet spectrophotometry method (UV method).

Non-reduced CE-SDS is a method for determining the purity of monoclonal antibodies using a capillary as a separation channel. In CE-SDS, protein migration is driven by the surface charge caused by SDS binding, which is proportional to the molecular weight of the protein. Since all SDS-protein complexes have similar mass-to-charge ratios, electrophoretic separation based on the size or hydrodynamic radius of the molecules can be achieved in the molecular sieve gel matrix of the capillary. This method has been widely used to monitor the purity of denatured intact antibodies. Generally, in non-reduced CE-SDS, the test sample is mixed with an SDS sample buffer and iodoacetamide. Then the mixture can be incubated at 68-72 °C for about 10-15 min and cooled to room temperature before the supernatant is centrifuged for analysis. The protein migration is detected using an ultraviolet detector to obtain an electrophoresis spectrogram. The purity of the antibody formulation can be calculated as the percentage of the IgG main peak area to the sum of all peak areas. For further description of the CE-SDS, see, e.g., Richard R. et al, Application of CE SDS gel in development of biopharmaceutical antibody-based products, Electrophoresis, 2008, 29, 3612-3620.

Size exclusion high performance liquid chromatography (SEC-HPLC) is another important method for the standardization and quality control of monoclonal antibodies. The method mainly separates molecules based on the differences in their size or hydrodynamic radius. Antibodies can be separated in three main forms by SEC-HPLC: high-molecular-weight species (HMMS), main peak (mainly antibody monomer), and low molecular-weight species (LMMS). The purity of antibody can be calculated as the percentage of the main peak area to the sum of all peak areas on the chromatogram. The percentage of antibody monomers in the formulation can be measured by SEC-HPLC, which gives information about the content of soluble aggregates and splices. For further description of SEC-HPLC, see, e.g., J. Pharm. Scien., 83:1645-1650, (1994); Pharm. Res., 11:485 (1994); J. Pharm. Bio. Anal., 15:1928 (1997); J. Pharm. Bio. Anal., 14:1133-1140 (1986). In addition, see also, e.g., R. Yang et al., High resolution separation of recombinant monoclonal antibodies by size exclusion ultra-high performance liquid chromatography (SE-UHPLC), Journal of Pharmaceutical and Biomedical Analysis (2015), http://dx.doi.org/10.1016/j.jpba.2015.02.032; and Alexandre Goyon et al., Protocols for the analytical characterization of therapeutic monoclonal antibodies, I- Non-denaturing chromatographic techniques, Journal of Chromatography, http://dx.doi.org/10.1016/j.jchromb.2017.05.010.

Imaged capillary isoelectric focusing (iCIEF) can be used to analyze the charge heterogeneity of monoclonal antibodies. This method can provide a quantitative distribution of charge variants. iCIEF separates molecules based on the difference in their charge in a pH gradient (apparent pI value). In iCIEF, the separation column is typically a short capillary (e.g., silica capillary, 5 cm length, 100 µm I.D.), the proteins are focused in the capillary column at high voltage, and the focus is monitored online in real time by a whole column imaging detection system operating at 280 nM. One advantage of this technique is that various charge variants of an antibody sample can be simultaneously recorded by the whole column detection system. Generally, in iCIEF, the sample is mixed with urea and an iCIEF buffer containing methylcellulose, pI molecular weight standards, and ampholytes. Then after the sample has been focused for a period of time, the absorbance at 280 nm can be measured using an iCIEF column, such as a ProtionSimple assembled iCIEF column, on an iCIEF analyzer, such as an iCE280 analyzer (Protein Simple, Santa Clara, CA.), to obtain a spectrum of the focused mAb charge variants. In the iCEIF spectrum, protein-related peaks eluted before the main peak (i.e., principal component) are classified as acidic components, while protein-related peaks eluted after the main peak are classified as basic components. The relative amounts of the principal component, acidic component and basic component can be expressed as a percentage to the total peak area. For further description of iCIEF, see, e.g., Salas-Solano O et al, Robustness of iCIEF methodology for the analysis of monoclonal antibodies: an interlaboratory study, J Sep Sci. 2012 Nov; 35(22):3124-9. doi: 10.1002/jssc.201200633. Epub 2012 Oct 15; and Dada OO et al, Characterization of acidic and basic variants of IgG1 therapeutic monoclonal antibodies based on non-denaturing IEF fractionation, Electrophoresis. 2015 Nov; 36(21-22):2695-2702. doi: 10.1002/elps.201500219. Epub 2015 Sep 18.

The charge variants of the antibody in the antibody formulation can also be determined by cation exchange high performance liquid chromatography (CEX-HPLC). In this method, peaks eluted from the CEX-HPLC column earlier than the retention time of the main peak are labeled as "acidic peaks", while those eluted from the CEX-HPLC column later than the retention time of the main peak are labeled as "basic peaks".

Accelerated stability studies can be used to check the stability of products, which facilitates the screening of stable pharmaceutical formulations. For example, formulation samples can be placed at an elevated temperature, e.g., about 40±2 °C and 25±2 °C for an accelerated stability study. The test indexes can include appearance, visible particles, protein content, turbidity, purity (SEC-HPLC and non-reduced CE-SDS) and charge variants (iCIEF and CEX-HPLC).

In addition, the efficacy or biological activity of the antibody can be detected. For example, the ability of an antibody in a formulation to bind to its antigenic molecules (CD47 molecule and PD-L1 molecule) can be tested. Various methods are known to those skilled in the art for quantifying the specific binding of an antibody to an antigen, such as immunoassay assays, e.g., ELISA.

The anti-CD47/PD-L1 bispecific antibody protein formulation disclosed herein is stable. In one embodiment, the purity of the anti-CD47/PD-L1 bispecific antibody protein in the antibody formulation disclosed herein is at least 85%, 90%, 91%, 92%, 93%, 94%, 95%, 96%, 97%, 98%, or 99% or more after storage at about 25 °C, 37 °C, 40 °C or 45 °C for at least 1 month or 2 months, e.g., after storage at 40±2 °C for 1 month, as determined by size exclusion chromatography or non-reduced CS-SDS. In one embodiment, at least 50%, preferably at least 55%, of the anti-CD47/PD-L1 bispecific antibody protein in the antibody formulation disclosed herein is in the non-basic and non-acidic forms (i.e., the main peak or main charge form) after storage at about 25 °C, 37 °C, 40 °C or 45 °C for at least 1 month or 2 months, e.g., after storage at 40±2 °C for 1 month, as determined by CEX-HPLC.

### IV. Use of Formulation

The antibody formulation comprising an anti-CD47/PD-L1 bispecific antibody protein disclosed herein can be used for treating, preventing or delaying various diseases or disorders associated with the SIRPα/CD47 signaling pathway and/or the PD1/PD-L1 signaling pathway. "Diseases or disorders associated with the SIRPα/CD47 signaling pathway" and/or "diseases or disorders associated with the PD1/PD-L1 signaling pathway" refer herein to diseases or disorders that can be treated (e.g., ameliorated) or prevented with the anti-CD47/PD-L1 bispecific antibody protein formulation disclosed herein. Any disease or disorder that can benefit from the treatment with the antibody formulation disclosed herein is suitable for the present invention.

In one aspect, the formulation comprising an anti-CD47/PD-L1 bispecific antibody protein disclosed herein can be used for preventing or treating various blood diseases and solid tumors in a subject, including but not limited to acute myeloid leukemia (AML), chronic myeloid leukemia, acute lymphocytic leukemia (ALL), non-Hodgkin's lymphoma (NHL), multiple myeloma (MM), lymphoma, breast cancer, gastric cancer, lung cancer, esophageal cancer, intestinal cancer, ovarian cancer, cervical cancer, renal cancer, pancreatic cancer, bladder cancer, neuroglioma, melanoma and other solid tumors. In addition, human stem cell implantation in a NOD mouse line can be enhanced by blocking the SIRPα/CD47 signaling pathway (WO 2009/046541), and thus the formulation comprising an anti-CD47/PD-L1 bispecific antibody protein disclosed herein also has potential benefits for human stem cell transplantation.

In another aspect, the formulation comprising an anti-CD47/PD-L1 bispecific antibody protein disclosed herein can be used for treating, preventing or diagnosing autoimmune diseases and inflammatory disorders mediated by SIRPα+ cells, e.g., allergic asthma or ulcerative colitis, in a subject. These disorders include acute and chronic inflammatory disorders, allergies and allergic diseases, autoimmune diseases, ischemic disorders, severe infections, and rejection for cell or tissue or organ transplant, including non-human tissue transplant (heterograft).

The present invention also provides use of the formulation disclosed herein in preparing a medicament for delivering the anti-CD47/PD-L1 bispecific antibody protein to a mammal, or for treating, preventing or ameliorating one or more of the diseases and disorders described above. Preferably, the mammal is a human.

The antibody formulation disclosed herein can be administered to a subject or a patient in a variety of pathways. For example, administration can be performed by infusion or by a syringe. Accordingly, in one aspect, the present invention provides a delivery device (e.g., a syringe) comprising the antibody formulation disclosed herein (e.g., a pre-filled syringe). The patient will receive an effective amount of the anti-CD47/PD-L1 bispecific antibody protein as the primary active ingredient, i.e., an amount sufficient to treat, ameliorate or prevent the disease or disorder of interest.

The therapeutic effect can include a reduction in physiological symptoms. The optimal effective amount and concentration of the antibody for any specific subject will depend on a variety of factors including the age, weight, health status and/or sex of the patient, the nature and extent of the disease, the activity of the specific antibody, its clearance by the body, as well as any possible other treatments administered in combination with the antibody formulation. For a specific case, the effective amount delivered can be determined within the judgment of a clinician. Depending on the indication to be treated, an effective dose can range from about 0.005 mg/kg body weight to about 50 mg/kg body weight, or from about 0.1 mg/kg body weight to about 20 mg/kg body weight. In this aspect, the use of known antibody-based drugs can provide some guidance. The dosage can be a single-dose regimen or a multi-dose regimen.

The following examples are described to assist in understanding the present invention. The examples are not intended to be and should not be interpreted in any way as limiting the protection scope of the present invention.

### Abbreviations

CE-SDS: capillary electrophoresis-sodium dodecyl sulfate
CEX-HPLC: cation exchange high performance liquid chromatography
ELISA: enzyme-linked immunosorbent assay
FLD-HPLC: HPLC with fluorescence detection
iCIEF: imaged capillary isoelectric focusing
SEC-HPLC: size exclusion high performance liquid chromatography

### Examples

In order to develop a formulation formula for long-term stable storage of a recombinant anti-cluster of differentiation 47 (CD47) and anti-programmed death ligand 1 (PD-L1) bispecific antibody injection and to ensure that the quality of the product is controllable over its shelf life (at least 24 months), a formula screening test is designed to investigate effect of different excipients on the stability of the anti-CD47/PD-L1 bispecific antibody formulation. The materials and methods used for the test are as follows:

### Materials and methods

### 1.1. Materials used in formulation research of present invention

| Name | Grade | Origi and brand | Catalog No. | Criteria |
|---|---|---|---|---|
| Histidine | Pharmaceutical grade | Ajinomoto, Shanghai | N/A | *Ch.P* (2015 edition) |
| Histidine hydrochloride | Pharmaceutical grade | Merck, Germany | N/A | *Ch.P* (2015 edition) |
| Arginine hydrochloride | Pharmaceutical grade | Ajinomoto, Shanghai | N/A | *Ch.P* (2015 edition) |
| Polysorbate-80 | Pharmaceutical grade | Well, Nanjing | Jiangsu MPA Approval No. F15423203 | *Ch.P* (2015 edition) |
| 2R vial | N/A | Schott, Suzhou | 1142144 | N/A |
| 13 mm rubber stopper | N/A | West, Singapore | 7002-4373 | N/A |
| 13 mm aluminum-plastic cap | N/A | West, India | 5413-3001 | N/A |

| | | | | |
|---|---|---|---|---|
| Note: N/A indicates "Not Applicable". | | | | |

### 1.2. Instruments and devices used in formulation research of present invention

| Name | Origin and brand | Model No. | No. |
|---|---|---|---|
| Constant temperature and humidity chamber | BINDER, Germany | KBF P 720 | PD-A1-069 |
| Biochemical incubator | Jinghong, Shanghai | SHP-150 | PD-A1-200 |
| Vortex mixer | VWR, USA | DVX-2500 | PD-A1-140 |
| Medical refrigerator | Haier, Qingdao | HYC-360 | PD-A1-166 |
| Ultra-low temperature refrigerator | Thermo, USA | 907 | PD-A1-175 |
| Clarity detector | Tianda Tianfa, Tianjin | YB-2 | PD-A1-033 |
| Ultraviolet-visible spectrophotometer | Shimadzu, Japan | UV-1800 | AS-A1-037 |
| pH meter | Mettler, Switzerland | FE20 | PD-A1-002 |
| Multi-channel microspectrophotometer | Thermo, USA | Nanodrop 8000 | PD-A1-052 |
| Benchtop refrigerated centrifuge | Thermo, USA | SL16R | PD-A1-082 |
| Clean bench | Antai Airtech, Suzhou | SW-CJ-2FD | QC-A1-011 |
| Multi-channel osmometer | ADVANCED | Model | BB-LA-00323 |

### 1.3. Test items and methods for formulation stability

The antibody formulation was tested for the following items: (1) the appearance and the presence of visible particles; (2) the protein content in the formulation determined by the ultraviolet method (UV method); (3) the purity of the antibody formulation determined by size exclusion chromatography (e.g., size exclusion high performance liquid chromatography (SEC-HPLC)) and expressed as the percentage of the monomer area to the sum of all peak areas; (4) the purity of the antibody formulation determined by reduced capillary electrophoresis-sodium dodecyl sulfate (reduced CE-SDS) and/or non-reduced capillary electrophoresis-sodium dodecyl sulfate (non-reduced CE-SDS) and expressed as the percentage of the monomer area to the sum of all peak areas; (5) charge variants in the antibody formulation determined by imaged capillary isoelectric focusing (iCIEF) and expressed as the percentage of the principal component, acidic component and basic component; and (6) the relative binding activity of the anti-CD47/PD-L1 bispecific antibody in the antibody formulation to antigens CD47 and PD-L1 determined by immunoassay, e.g., direct ELISA.

### Detection of visible particles

The visible particles in the sample were detected using a clarity detector (model No. YB-2, Tianda Tianfa, Tianjin) according to the method described in the National Pharmacopoeia Committee, the Pharmacopoeia of the People's Republic of China (2015 edition, volume IV General Rules 0904 "Test for Visible Particles"), Beijing, China Medical Science Press, 2015.

### Determination of protein content

The protein content in the sample was determined using an ultraviolet spectrophotometer (model No. UV-1800, Shimadzu, Japan).

### Purity (SEC-HPLC)

Separation was performed using a size exclusion chromatographic column, wherein the mobile phase was phosphate buffer (3.12 g of sodium dihydrogen phosphate dihydrate, 8.77 g of sodium chloride and 34.84 g of arginine were dissolved in ultra-pure water, and the pH of the solution was adjusted to 6.8 by hydrochloric acid, and the volume was made up to 1000 mL), the chromatographic column protection solution was 0.05% (w/v) NaN₃, the injection volume was 50 µL, the flow rate was 0.5 mL/min, the acquisition time was 30 min, the column temperature was 25 °C, and the detection wavelength was 280 nm. A sample was diluted to 2 mg/mL with ultra-pure water to be used as a sample solution. A formulation buffer was diluted in the same manner as described above to prepare a blank solution. 50 µL of each of the blank solution and the sample solution was injected into a liquid chromatograph, and the detection was started.

### Purity (reduced CE-SDS)

The purity was detected by capillary gel electrophoresis. The capillary tube was an uncoated capillary tube and had an inner diameter of 50 µm, a total length of 30.2 cm and an effective length of 20.2 cm. Before electrophoresis, the capillary column was washed with 0.1 mol/L sodium hydroxide, 0.1 mol/L hydrochloric acid, ultra-pure water and electrophoresis gel at 70 psi. A sample was diluted to 2.0 mg/mL with an appropriate amount of ultra-pure water, and 50 µL of the diluted sample was added into a 1.5 mL centrifuge tube. To the centrifuge tube were then added 45 µL of sample buffer at pH 6.5 (0.32 g of citric acid monohydrate and 2.45 g of disodium hydrogen phosphate dodecahydrate were dissolved in 45 mL of ultra-pure water, and the volume was made up to 50 mL to prepare a citric acid-phosphate buffer; 200 µL of the buffer was precisely measured out, and then 80 µL of 10% (w/v) sodium dodecyl sulfate solution was added; the volume was made up to 1 mL with water, and then the mixture was well mixed to obtain the sample buffer), 1 µL of internal standard (10 kDa protein, 5 mg/mL) (Beckman Coulter, Catalog No. 390953) and 5 µL of β-mercaptoethanol. The resulting mixture was well mixed, heated at 70±2 °C for 10±2 min, and then cooled to room temperature and transferred to a sample bottle to be used as a sample solution. A formulation buffer of the same volume as the sample was processed by the same method as above to prepare the blank solution. Conditions for sample injection: -5 kV for 20 s; separation voltage: -15 kV for 35 min. The capillary column temperature was controlled at 25 °C, and the detection wavelength was 220 nm.

### Purity (non-reduced CE-SDS method)

The purity was detected by capillary gel electrophoresis. The capillary tube was an uncoated capillary tube and had an inner diameter of 50 µm, a total length of 30.2 cm and an effective length of 20.2 cm. Before electrophoresis, the capillary column was washed with 0.1 mol/L sodium hydroxide, 0.1 mol/L hydrochloric acid, ultra-pure water and electrophoresis gel at 70 psi. A sample was diluted to 2.0 mg/mL with an appropriate amount of ultra-pure water, and 50 µL of the diluted sample was added into a 1.5 mL centrifuge tube. To the centrifuge tube were then added 45 µL of sample buffer at pH 6.5 (0.32 g of citric acid monohydrate and 2.45 g of disodium hydrogen phosphate dodecahydrate were dissolved in 45 mL of ultra-pure water, and the volume was made up to 50 mL to prepare a citric acid-phosphate buffer; 200 µL of the buffer was precisely measured out, and then 80 µL of 10% (w/v) sodium dodecyl sulfate solution was added; the volume was made up to 1 mL with water, and then the mixture was well mixed to obtain the sample buffer), 1 µL of internal standard (10 kDa protein, 5 mg/mL) (Beckman Coulter, Catalog No. 390953) and 5 µL of 250 mmol/L NEM solution (62 mg of *N*-ethylmaleimide was dissolved in 2 mL of ultra-pure water). The resulting mixture was well mixed, heated at 70±2 °C for 10±2 min, and then cooled to room temperature and transferred to a sample bottle to be used as a sample solution. A formulation buffer of the same volume as the sample was processed by the same method as above to prepare the blank solution. Conditions for sample injection: -5 kV for 20 s; separation voltage: -15 kV for 35 min. The capillary column temperature was controlled at 25 °C, and the detection wavelength was 220 nm.

### Charge variants (iCIEF method)

The charge variants were detected by imaged capillary isoelectric focusing (iCIEF). The inner diameter of the capillary tube was 100 µm, and the total length was 5 cm. The capillary column was rinsed with 0.5% methylcellulose solution (hereinafter abbreviated as MC solution) and ultra-pure water before electrophoresis. The sample was injected in vacuum for 55 s, the pre-focusing was conducted at 1.5 kV for 1 min, the focusing was conducted at 3 kV for 8 min, the sample injection time was 55 s, the temperature of the sample tray was 10 °C, the capillary column temperature was room temperature, and the detection wavelength was 280 nm. The cathodic stabilizer was 500 mmol/L arginine, and the anodic stabilizer was 200 mmol/L iminodiacetic acid. 3 mol/L urea was added to improve the protein solubility, and 0.5% MC solution was added to decrease the adhesion between the protein and the capillary. The sample was diluted to 0.5 mg/mL with water, and then 20 µL of the diluted sample solution was well mixed with 83 µL of a premixed solution to prepare a sample solution. The same procedures were performed using the formulation buffer to prepare a blank solution.

### Relative binding activity (direct ELISA)

Streptavidin (Thermo, Catalog No.: 21125) was diluted to 1 µg/mL with 1×PBS, and then coated on a 96-well microplate at 100 µL/well at 37 °C for 2 h. After being washed, the plate was blocked with a blocking solution (5% FBS, 300 µL/well) at 37 °C for 2 h. Biotinylated antigen (for detection of the relative binding activity of an anti-CD47 end of an anti-CD47/PD-L1 bispecific antibody to CD47, human CD47 protein (His-tag) from Beijing Sino Biological (catalog No.: 12283-H08H-200) was used; for detection of the relative binding activity of an anti-PD-L1-end of an anti-CD47/PD-L1 bispecific antibody to PD-L1, recombinant human PDL1/CD274 protein from ACRO BIOSYSTEMS (catalog No.: PD1-H5229-1MG) was used) was diluted to 0.5 µg/mL with 1×PBS, and then coated on a 96-well microplate at 100 µL/well at 37 °C for 0.5 h. 2% FBS was added at 100 µL/well to dilute the anti-CD47/PD-L1 bispecific antibody to 40 µg/mL, and a 4-fold serial dilution was performed to obtain 12 concentrations (0.01-10000 ng/mL). The serially diluted sample was added to the washed microplate at 100 µL/well and incubated at 37 °C for 30 min in a thermostatic incubator. After the plate was washed, HRP-conjugated goat anti-human IgG-Fc fragment (BETHYL, USA, catalog No. A80-104P) diluted with 2% FBS was added as a secondary antibody (30000-fold dilution, 100 µL/well) for reaction at 37 °C for 20 min. After the plate was washed, 100 µL of TMB chromogenic solution was added. After 10 min of chromogenic reaction, 1 mol/L H₂SO₄ was added at 100 µL/well to terminate the reaction. The OD value at 450 nm was measured using 620 nm as a reference wavelength. By taking the concentration values of the sample at all concentration gradients as an abscissa and the OD_{450 mn}-OD_{620 nm} values of the sample at all concentration gradients as an ordinate, EC₅₀ values were calculated by Prism four-parameter fitting to reflect the binding activity of the antibody to each antigen.

### Example 1: Preparation and Purification of Anti-CD47/PD-L1 Bispecific Antibody

The anti-CD47/PD-L1 bispecific antibody Kh2NF-PC was recombinantly expressed in HEK293 cells (purchased from INVITROGEN) and was purified, as described in PCT/CN2018/123886. The anti-CD47/PD-L1 bispecific antibody Kh2NF-PC antibody consists of 3 polypeptide chains, each polypeptide chain having the following amino acid sequence from N-terminus to C-terminus:
Peptide chain #1: 
QVQLQESGPGLVKPSETLSLTCTVSGGSIEHYYWSWIRQPPGKGLEWIGYIYYSGSTNYNPS
LKSRVTISVDTSKNQFSLKLSSVTAADTAVYYCARGKTGSAAWGQGTLVTVSSASTKGPS
VFPLAPSSKSTSGGTAALGCLVKDYFPEPVTVSWNSGALTSGVHTFPAVLQSSGLYSLSSV
VTVPSSSLGTQTYICNVNHKPSNTKVDKKVEPKSCDKTHTCPPCPAPEAAGGPSVFLFPPKP
KDTLMISRTPEVTCVVVDVSHEDPEVKFNWYVDGVEVHNAKTKPREEQYNSTYRVVSVL
TVLHQDWLNGKEYKCKVSNKALPAPIEKTISKAKGQPREPQVYTLPPCRDELTKNQVSLW
CLVKGFYPSDIAVEWESNGQPENNYKTTPPVLDSDGSFFLYSKLTVDKSRWQQGNVFSCS
VMHEALHNHYTQKSLSLSPG (SEQ ID NO: 1)
 wherein, the peptide chain #1 comprises: the following VH amino acid sequence derived from the anti-CD47 antibody ADI29341: 
QVQLQESGPGLVKPSETLSLTCTVSGGSIEHYYWSWIRQPPGKGLEWIGYIYYSGSTNYNPS
LKSRVTISVDTSKNQFSLKLSSVTAADTAVYYCARGKTGSAAWGQGTLVTVSS (SEQ ID
NO: 2);
 the following CH1 amino acid sequence derived from human IgG1 at the C-terminus of the VH amino acid sequence:
   ASTKGPSVFPLAPSSKSTSGGTAALGCLVKDYFPEPVTVSWNSGALTSGVHTFPAVLQSSG LYSLSSVVTVPSSSLGTQTYICNVNHKPSNTKVDKKVEPKSCDKTHT (SEQ ID NO: 6); and
   the following Fc region amino acid sequence derived from human IgG1 at the C-terminus of the CH1 amino acid sequence: 
CPPCPAPEAAGGPSVFLFPPKPKDTLMISRTPEVTCVVVDVSHEDPEVKFNWYVDGVEVHN
AKTKPREEQYNSTYRVVSVLTVLHQDWLNGKEYKCKVSNKALPAPIEKTISKAKGQPREP
QVYTLPPCRDELTKNQVSLWCLVKGFYPSDIAVEWESNGQPENNYKTTPPVLDSDGSFFLY
SKLTVDKSRWQQGNVFSCSVMHEALHNHYTQKSLSLSPG (SEQ ID NO: 7)
Peptide chain #2: 
DIQMTQSPSSVSASVGDRVTITCRASQGISRWLAWYQQKPGKAPKLLIYAASSLQSGVPSRF
SGSGSGTDFTLTISSLQPEDFATYYCQQTVSFPITFGGGTKVEIKRTVAAPSVFIFPPSDEQLK
SGTASVVCLLNNFYPREAKVQWKVDNALQSGNSQESVTEQDSKDSTYSLSSTLTLSKADY
EKHKVYACEVTHQGLSSPVTKSFNRGEC (SEQ ID NO: 8)
 wherein, the peptide chain #2 comprises: the following VL amino acid sequence derived from the anti-CD47 antibody ADI29341:
   DIQMTQSPSSVSASVGDRVTITCRASQGISRWLAWYQQKPGKAPKLLIYAASSLQSGVPSRF SGSGSGTDFTLTISSLQPEDFATYYCQQTVSFPITFGGGTKVEIK (SEQ ID NO: 9); and
   the following amino acid sequence of human kappa light chain constant region (CL) at the C-terminus of the VL amino acid sequence: 

 (SEQ ID NO: 13)
Peptide chain #3: 
QVQLQESGGGLVQPGGSLRLSCAASAYTISRNSMGWFRQAPGKGLEGVAAIESDGSTSYS
DSVKGRFTISLDNSKNTLYLEMNSLRAEDTAVYYCAAPKVGLGPRTALGHLAFMTLPALN
YWGQGTLVTVSSGGGGSGGGGSGGGGSGGGGSQVQLQESGGGLVQPGGSLRLSCAASAY
TISRNSMGWFRQAPGKGLEGVAAIESDGSTSYSDSVKGRFTISLDNSKNTLYLEMNSLRAE
DTAVYYCAAPKVGLGPRTALGHLAFMTLPALNYWGQGTLVTVSSDKTHTCPPCPAPEAA
GGPSVFLFPPKPKDTLMISRTPEVTCVVVDVSHEDPEVKFNWYVDGVEVHNAKTKPREEQ
YNSTYRVVSVLTVLHQDWLNGKEYKCKVSNKALPAPIEKTISKAKGQPREPQVCTLPPSRD
ELTKNQVSLSCAVKGFYPSDIAVEWESNGQPENNYKTTPPVLDSDGSFFLVSKLTVDKSRW
QQGNVFSCSVMHEALHNHYTQKSLSLSPG (SEQ ID NO: 14)
 wherein, the peptide chain #3 comprises: the following amino acid sequence of the first anti-PD-L1 VHH and the second anti-PD-L1 VHH 
QVQLQESGGGLVQPGGSLRLSCAASAYTISRNSMGWFRQAPGKGLEGVAAIESDGSTSYS
DSVKGRFTISLDNSKNTLYLEMNSLRAEDTAVYYCAAPKVGLGPRTALGHLAFMTLPALN
YWGQGTLVTVSS (SEQ ID NO: 16);
 the linker peptide amino acid sequence between the amino acid sequences of the first anti-PD-L1 VHH and the second anti-PD-L1 VHH: GGGGSGGGGSGGGGSGGGGS (SEQ ID NO: 20); and
   the following Fc region amino acid sequence derived from human IgG1 at the C-terminus of the amino acid sequence of the second anti-PD-L1 VHH

DKTHTCPPCPAPEAAGGPSVFLFPPKPKDTLMISRTPEVTCVVVDVSHEDPEVKFNWYVDG
VEVHNAKTKPREEQYNSTYRVVSVLTVLHQDWLNGKEYKCKVSNKALPAPIEKTISKAKG
QPREPQVCTLPPSRDELTKNQVSLSCAVKGFYPSDIAVEWESNGQPENNYKTTPPVLDSDG
SFFLVSKLTVDKSRWQQGNVFSCSVMHEALHNHYTQKSLSLSPG (SEQ ID NO: 21).

### Example 2: Test for Effect of pH on Stability of Formulation (I)

This example investigates the stability of formulations comprising an anti-CD47/PD-L1 bispecific antibody at pH 5.0-6.5. A total of 4 pH values were designed, namely 5.0, 5.5, 6.0 and 6.5.

### 2.1. Experimental procedures

10 mM histidine-5% (w/v) sorbitol buffer was prepared, and the pH was adjusted to 5.0, 5.5, 6.0 and 6.5 with diluted hydrochloric acid. Purified anti-CD47/PD-L1 bispecific antibody Kh2NF-PC protein (7.3 mg/mL) was exchanged into solutions of the different pH values by ultrafiltration. The content of the bispecific antibody protein in the samples was adjusted to about 100.0 mg/mL after the exchange, and then polysorbate-80 was added until its final concentration was 0.70 mg/mL. The solutions were filtered and aliquoted into 2R vials, followed by plugging and capping. The stability of the samples was investigated at 40±2 °C and the specific experimental scheme is shown in Table 1.

**Table 1. Experimental scheme**

| Experimental conditions | Sampling time points | | | | Test items |
|---|---|---|---|---|---|
| | Day 0 | Week 1 | Week 2 | Month 1 | |
| 40±2 °C | x | x | x | x | Appearance, visible particles, protein content, purity (SEC-HPLC and CE-SDS), charge variants (iCIEF) and relative binding activity (direct ELISA) |
| | | x | x | x | |

| | | | | | |
|---|---|---|---|---|---|
| Note: (1) x represents that sampling is performed at this time point. (2) After sampling at these time points, the obtained samples were first put into an ultra-low temperature refrigerator and frozen for later detection, and then thawed for detection as required. | | | | | |

### 2.2. Criteria for determination

According to the knowledge about the product and the precision of the instrument and the method, criteria for determining that the sample test indexes have not changed compared to initial values were set to determine whether the sample has changed, as detailed in Table 2.

**Table 2. Criteria for determining absence of quality change**

| Test items | Criteria for determining absence of change |
|---|---|
| Appearance (Observation) | Clear to slightly opalescent, colorless to pale yellow liquid, no particles |
| Visible particles (Test for visible particles) | Conforms to the General Rule 0904 of the Pharmacopoeia of the People's Republic of China (2015 edition, volume IV) |
| Protein content (UV method) | Change rate ≤ 10% |
| Purity (SEC-HPLC) | Change in main peak purity ≤ 1% |
| Purity (reduced CE-SDS) | Change in main peak purity ≤ 2% |
| Purity (non-reduced CE-SDS method) | Change in main peak purity ≤ 2% |
| Charge variants (iCIEF method) | Change in the principal component and the acidic and basic components ≤ 2% |
| Charge variants (CEX-HPLC) | Change in the principal component and the acidic and basic components ≤ 2% |
| Relative binding activity (direct ELISA) | Should be 70-130% |

### 2.3. Experimental results of formula screening test (I)

### (1) Appearance and visible particles

After storage at 40±2 °C for one month, the appearance of the samples at pH 5.0, 5.5 and 6.0 showed different degrees of turbidness and precipitation, and only the pH 6.5 sample was up to standard in terms of appearance and visible particles.

### (2) Protein content

Detection results of the protein content of the samples at pH 5.0, 5.5, 6.0 and 6.5 after storage at 40±2 °C for different time periods are shown in Table 3. The results show that the protein content of the pH 6.5 sample didn't change significantly after storage at 40±2 °C for 1 month.

**Table 3. Protein content of samples at pH 5.0, 5.5, 6.0 and 6.5 after storage at 40±2 °C for different time periods (UV method, mg/mL)**

| Sample name | Time | | | |
|---|---|---|---|---|
| | Day 0 | Week 1 | Week 2 | Month 1 |
| pH 5.0 | 99.3 | N/A | N/A | N/A |
| pH 5.5 | 105.8 | N/A | N/A | N/A |
| pH 6.0 | 104.4 | 103.6 | N/A | N/A |
| pH 6.5 | 103.9 | 105.0 | 100.9 | 103.1 |

| | | | | |
|---|---|---|---|---|
| Note: N/A indicates that no detection is performed for the sample as its appearance is not up to standard. | | | | |

### (3) Purity

After storage at 40±2 °C for different time periods, the protein purity of the samples at pH 5.0, 5.5, 6.0 and 6.5 was determined by SEC-HPLC. The results are shown in Table 4. The results show that the protein purity of the pH 6.5 sample decreased by 4.1% compared to that on day 0 after investigation at 40±2 °C for 1 month.

**Table 4. Protein purity of samples determined by SEC-HPLC (%)**

| Sample name | Time | | | |
|---|---|---|---|---|
| | Day 0 | Week 1 | Week 2 | Month 1 |
| pH 5.0 | 99.2 | N/A | N/A | N/A |
| pH 5.5 | 99.0 | N/A | N/A | N/A |
| pH 6.0 | 98.9 | 97.2 | N/A | N/A |
| pH 6.5 | 98.6 | 95.9 | 95.1 | 94.5 |

| | | | | |
|---|---|---|---|---|
| Note: N/A indicates that no detection is performed for the sample as its appearance is not up to standard. | | | | |

After storage at 40±2 °C for different time periods, the protein purity of the samples at pH 5.0, 5.5, 6.0 and 6.5 was determined by non-reduced CE-SDS and reduced CE-SDS. The results are shown in Table 5 and Table 6. The results show that the protein purity of the pH 6.5 sample decreased by 7.7% and 3.7% respectively for the two methods compared to that on day 0 after investigation at 40±2 °C for 1 month.

**Table 5. Protein purity of samples determined by non-reduced CE-SDS (%)**

| Sample name | Time | | | |
|---|---|---|---|---|
| | Day 0 | Week 1 | Week 2 | Month 1 |
| pH 5.0 | 97.0 | N/A | N/A | N/A |
| pH 5.5 | 97.2 | N/A | N/A | N/A |
| pH 6.0 | 97.0 | 95.9 | N/A | N/A |
| pH 6.5 | 97.0 | 94.9 | 93.4 | 89.3 |

| | | | | |
|---|---|---|---|---|
| Note: N/A indicates that no detection is performed for the sample as its appearance is not up to standard. | | | | |

**Table 6. Protein purity of samples determined by reduced CE-SDS (%)**

| Sample name | Time | | | |
|---|---|---|---|---|
| | Day 0 | Week 1 | Week 2 | Month 1 |
| pH 5.0 | 98.9 | N/A | N/A | N/A |
| pH 5.5 | 98.9 | N/A | N/A | N/A |
| pH 6.0 | 98.8 | 99.3 | N/A | N/A |
| pH 6.5 | 98.6 | 99.2 | 97.8 | 94.9 |

| | | | | |
|---|---|---|---|---|
| Note: N/A indicates that no detection is performed for the sample as its appearance is not up to standard. | | | | |

### (4) Charge variants

After storage at 40±2 °C for different time periods, the charge variants of the samples at pH 5.0, 5.5, 6.0 and 6.5 were determined by iCIEF. The results are shown in Table 7. The results show that the principal component and the acidic component of the pH 6.5 sample changed significantly after investigation at 40±2 °C for 1 month. Compared with values on day 0, the acidic component increased from 36.6% to 60.1%, an increase of 23.5%; the principal component decreased from 62.5% to 39.1%, a decrease of 23.4%; the basic component did not change significantly.

**Table 7. Charge variants of samples determined by iCIEF (%)**

| Sample name | | Time | | | |
|---|---|---|---|---|---|
| | | Day 0 | Week 1 | Week 2 | Month 1 |
| pH 5.0 | Acidic component | 36.2 | N/A | N/A | N/A |
| | Princi pal component | 62.8 | N/A | N/A | N/A |
| | Basic component | 1.0 | N/A | N/A | N/A |
| pH 5.5 | Acidic component | 36.2 | N/A | N/A | N/A |
| | Princi pal component | 62.8 | N/A | N/A | N/A |
| | Basic component | 1.0 | N/A | N/A | N/A |
| pH 6.0 | Acidic component | 37.1 | N/A | N/A | N/A |
| | Princi pal component | 61.8 | N/A | N/A | N/A |
| | Basic component | 1.1 | N/A | N/A | N/A |
| pH 6.5 | Acidic component | 36.6 | 43.3 | 50.0 | 60.1 |
| | Princi pal component | 62.5 | 55.7 | 49.0 | 39.1 |
| | Basic component | 0.9 | 1.1 | 1.0 | 0.8 |

| | | | | | |
|---|---|---|---|---|---|
| Note: N/A indicates that no detection is performed for the sample as its appearance is not up to standard. | | | | | |

### (5) Relative binding activity

After storage at 40±2 °C for different time periods, the relative binding activity of the samples at pH 5.0, 5.5, 6.0 and 6.5 were determined by direct ELISA. The results are shown in Table 8. The results show that, in the pH 6.5 sample, the relative binding activities of the anti-CD47 end and the anti-PD-L1 end of the protein for CD47 and PD-L1 respectively didn't change after investigation at 40±2 °C for 1 month.

**Table 8. Relative binding activity of samples determined by direct ELISA (%)**

| Sample name | | Time | | | |
|---|---|---|---|---|---|
| | | Day 0 | Week 1 | Week 2 | Month 1 |
| pH 5.0 | Anti-CD47 end | N/A¹ | N/A² | N/A² | N/A¹ |
| | Anti PD-L 1 end | N/A¹ | N/A² | N/A² | N/A¹ |
| pH 5.5 | Anti-CD47 end | N/A¹ | N/A² | N/A² | N/A¹ |
| | Anti PD-L 1 end | N/A¹ | N/A² | N/A² | N/A¹ |
| pH 6.0 | Anti-CD47 end | N/A¹ | N/A² | N/A² | N/A¹ |
| | Anti PD-L 1 end | N/A¹ | N/A² | N/A² | N/A¹ |
| pH 6.5 | Anti-CD47 end | 127 | N/A² | N/A² | 110 |
| | Anti PD-L 1 end | 79 | N/A² | N/A² | 95 |

| | | | | | |
|---|---|---|---|---|---|
| Note: N/A¹ indicates that no detection is performed for the sample as its appearance is not up to standard; N/A² indicates that the test item is not set. | | | | | |

The results of the above experiment show that for formulations at pH 5.0, 5.5, 6.0 and 6.5, the anti-CD47/PD-L1 bispecific antibody protein (e.g., Kh2NF-PC protein) was stable in pH 6.5 formulation. To investigate the stability of the formulation at a pH around 6.5, further experiment was performed.

### Example 3: Test for Effect of pH on Stability of Formulation (II)

This example investigates the effect of pH 6.2-7.0 on stability of protein in an anti-CD47/PD-L1 bispecific antibody formulation, and a total of 5 pH values were designed, namely 6.2, 6.4, 6.5, 6.8 and 7.0.

### 3.1. Experimental procedures

The specific procedures were the same as in "2.1. Experimental procedures" in Example 2, except that the pH values were different.

### 3.2. Criteria for determining

See Table 2 in Example 2.

### 3.3. Experimental results

### (1) Appearance and visible particles

After storage at 40±2 °C for one month, only the pH 6.2 sample showed a milk white appearance, and the samples at pH 6.4, 6.5, 6.8 and 7.0 were all up to standard in terms of appearance and visible particles.

### (2) Protein content

Detection results of the protein content of the samples at pH 6.4, 6.5, 6.8 and 7.0 after storage at 40±2 °C for different time periods are shown in Table 9. The results show that the protein content of the pH 6.4, 6.5, 6.8 and 7.0 samples didn't change significantly after storage at 40±2 °C for 1 month.

**Table 9. Protein content of samples at pH 6.4, 6.5, 6.8 and 7.0 after storage at 40±2 °C for different time periods (UV method, mg/mL)**

| Sample name | Time | | | |
|---|---|---|---|---|
| | Day 0 | Week 1 | Week 2 | Month 1 |
| pH 6.4 | 101.5 | N/A | 100.4 | 102.6 |
| pH 6.5 | 99.1 | N/A | 98.5 | 103.1 |
| pH 6.8 | 92.9 | N/A | 96.6 | 91.5 |
| pH 7.0 | 95.8 | N/A | 92.9 | 92.6 |

| | | | | |
|---|---|---|---|---|
| Note: N/A indicates that the test item is not set. | | | | |

### (3) Purity

After storage at 40±2 °C for different time periods, the protein content of the samples at pH 6.4, 6.5, 6.8 and 7.0 was determined by SEC-HPLC. The results are shown in Table 10, and the trend of change in purity is shown in FIG. 2. The results show that the purity of the samples at pH 6.4, 6.5, 6.8 and 7.0 decreased to some extent, namely by 3.0%, 3.7%, 4.9% and 5.6%, respectively, compared to that on day 0 after investigation at 40±2 °C for 1 month.

**Table 10. Protein purity of samples determined by SEC-HPLC %**

| Sample name | Time | | | |
|---|---|---|---|---|
| | Day 0 | Week 1 | Week 2 | Month 1 |
| pH 6.4 | 97.5 | N/A | 95.2 | 94.5 |
| pH 6.5 | 97.4 | N/A | 94.7 | 93.7 |
| pH 6.8 | 97.3 | N/A | 93.8 | 92.4 |
| pH 7.0 | 97.1 | N/A | 93.3 | 91.5 |

After storage at 40±2 °C for different time periods, the protein content of the samples at pH 6.4, 6.5, 6.8 and 7.0 was determined by non-reduced CE-SDS. The results are shown in Table 11, and the trend of change in purity is shown in FIG. 3. The results show that the purity of the samples at pH 6.4, 6.5, 6.8 and 7.0 decreased by 7.0%, 6.1%, 6.7% and 7.3% respectively compared to that on day 0 after investigation at 40±2 °C for 1 month.

**Table 11. Protein purity of samples determined by non-reduced CE-SDS (%)**

| Sample name | Time | | | |
|---|---|---|---|---|
| | Day 0 | Week 1 | Week 2 | Month 1 |
| pH 6.4 | 98.4 | N/A | 95.2 | 91.4 |
| pH 6.5 | 98.4 | N/A | 95.4 | 92.3 |
| pH 6.8 | 98.4 | N/A | 95.0 | 91.7 |
| pH 7.0 | 98.2 | N/A | 94.9 | 90.9 |

| | | | | |
|---|---|---|---|---|
| Note: N/A indicates that the test item is not set. | | | | |

### (4) Charge variants

After storage at 40±2 °C for different time periods, the charge variants of the samples at pH 6.4, 6.5, 6.8 and 7.0 were determined by iCIEF. The results are shown in Table 12, and the trend of change in purity is shown in FIG. 4. The results show that the principal component and the acidic component of the samples at different pH values changed after investigation at 40±2 °C for 1 month. The higher the pH, the faster the principal component decreased and the faster the acidic component increased.

**Table 12. Charge variants of samples determined by iCIEF (%)**

| Sample name | | Time | | | |
|---|---|---|---|---|---|
| | | Day 0 | Week 1 | Week 2 | Month 1 |
| pH 6.4 | Acidic component | 34.7 | N/A | 46.0 | 54.2 |
| | Princi pal component | 64.3 | N/A | 52.7 | 44.8 |
| | Basic component | 1.0 | N/A | 1.2 | 1.0 |
| pH 6.5 | Acidic component | 34.1 | N/A | 48.7 | 56.9 |
| | Princi pal component | 64.7 | N/A | 50.1 | 42.3 |
| | Basic component | 1.2 | N/A | 1.2 | 0.8 |
| pH 6.8 | Acidic component | 34.5 | N/A | 51.1 | 62.2 |
| | Princi pal component | 64.5 | N/A | 47.8 | 37.2 |
| | Basic component | 1.0 | N/A | 1.1 | 0.7 |
| pH7.0 | Acidic component | 34.1 | N/A | 53.3 | 65.5 |
| | Princi pal component | 64.8 | N/A | 45.7 | 33.9 |
| | Basic component | 1.1 | N/A | 1.0 | 0.6 |

| | | | | | |
|---|---|---|---|---|---|
| Note: N/A indicates that the test item is not set. | | | | | |

### (5) Relative binding activity

After storage at 40±2 °C for different time periods, the relative binding activity of the samples at pH 6.4, 6.5, 6.8 and 7.0 were determined by direct ELISA. The results are shown in Table 13. The results show that, in the samples at pH 6.4, 6.5 and 7.0, the relative binding activities of the anti-CD47 end and the anti-PD-L1 end of the protein for CD47 and PD-L1 respectively didn't change significantly after investigation at 40±2 °C for 1 month.

**Table 13. Relative binding activity of samples determined by direct ELISA (%)**

| Sample name | | Time | | | |
|---|---|---|---|---|---|
| | | Day 0 | Week 1 | Week 2 | Month 1 |
| pH 6.4 | Anti-CD47 end | 94 | N/A | N/A | 96 |
| | Anti PD-L 1 end | 124 | N/A | N/A | 122 |
| pH 6.5 | Anti-CD47 end | 77 | N/A | N/A | 97 |
| | Anti PD-L 1 | 101 | N/A | N/A | 107 |

| Sample name | | Time | | | |
|---|---|---|---|---|---|
| | | Day 0 | Week 1 | Week 2 | Month 1 |
| | end | | | | |
| pH 6.8 | Anti-CD47 end | N/A | N/A | N/A | N/A |
| | Anti PD-L 1 end | N/A | N/A | N/A | N/A |
| pH 7.0 | Anti-CD47 end | 94 | N/A | N/A | 87 |
| | Anti PD-L 1 end | 103 | N/A | N/A | 121 |

| | | | | | |
|---|---|---|---|---|---|
| Note: N/A indicates that the test item is not set. | | | | | |

The test results of the effect of pH on the stability of formulation in Examples 2 and 3 show that: when the anti-CD47/PD-L1 bispecific antibody (e.g., Kh2NF-PC) protein at pH 5.0-6.2 is stored at 40±2 °C for one month, the sample becomes turbid or milk white precipitate is present as time goes by; when the sample at pH 6.4-7.0 is stored at 40±2 °C for one month, the sample is up to standard in terms of the appearance and visible particles, the protein content doesn't change significantly, and the relative binding activity for CD47 and PD-L1 is not changed remarkably. Thus, in the following examples, pH 6.5 was selected from pH 6.4-7.0 for subsequent experiments.

### Example 4. Formula Screening Test

### 4.1. Stabilizer screening test

Different stabilizers (sorbitol, sucrose, trehalose, arginine hydrochloride, etc.) were investigated for their effect on stability of a formulation comprising anti-CD47/PD-L1 bispecific antibody.

### 4.1.1 Procedures for stabilizer screening

A total of 5 formulas were designed and detailed in Table 14. Buffers of the formulas were prepared according to Table 14, and the anti-CD47/PD-L1 bispecific antibody (Kh2NF-PC) protein (3.6 mg/mL) was exchanged into respective formula solution by ultrafiltration. The protein content in each formula solution was adjusted to about 100.0 mg/mL after the exchange, and then polysorbate-80 was added until the final concentration of polysorbate-80 was 0.20 mg/mL. The solutions were filtered and aliquoted into vials, followed by plugging and capping. The stability of the samples was investigated at 40±2 °C and the specific scheme is shown in Table 15. The test indexes include appearance, visible particles, protein content, purity (SEC-HPLC) and charge variants (iCIEF).

**Table 14. Information about formulas selected for stabilizer screening test**

| No. | Formula information |
|---|---|
| Formula 1 | 20 mM histidine, 5% sorbitol, 0.02% polysorbate-80, pH 6.0 |
| Formula 2 | 20 mM histidine, 5% sorbitol, 0.02% polysorbate-80, pH 6.5 |
| Formula 3 | 20 mM histidine, 8% trehalose, 0.02% polysorbate-80, pH 6.5 |
| Formula 4 | 20 mM histidine, 180 mM arginine hydrochloride, 0.02% polysorbate-80, pH 6.5 |
| Formula 5 | 20 mM histidine, 4% sucrose, 100 mM arginine hydrochloride, 0.02% polysorbate-80, pH 6.5 |

| | |
|---|---|
| Note: the % in the table refer to % w/v; the same applies hereinafter. | |

**Table 15. Stability investigation scheme**

| Experimental conditions | Sampling time points | | | | Test items |
|---|---|---|---|---|---|
| | Day 0 | Week 1 | Week 2 | Week 4 | |
| 40±2 °C | x | x | x | x | Appearance, visible particles, protein content, purity (SEC-HPLC and CE-SDS) and charge variants (iCIEF) |
| | | x | x | x | |

| | | | | | |
|---|---|---|---|---|---|
| Notes: (1) x represents that sampling is performed at this time point. (2) After sampling at the above time points, the obtained samples were first put into an ultra-low temperature refrigerator and frozen for later detection, and then thawed for detection as required. | | | | | |

### 4.1.2. Criteria for determination

See Table 2 in Example 2 for the specific criteria.

### 4.1.3. Stabilizer screening test

### (1) Appearance and visible particles

The formula samples were observed at 40±2 °C until week 4; turbidness or precipitation were observed in formula 1 sample after investigation for one week, and other formula samples were up to standard in terms of appearance and visible particles.

### (2) Protein content

The formula samples were observed at 40±2 °C until week 4, and the results of protein content are shown in Table 16. As can be seen from Table 16, the protein content of formula 2, formula 3, formula 4 and formula 5 samples did not change after storage at 40±2 °C for 4 weeks.

**Table 16. Protein content results in stabilizer screening test (UV method, mg/mL)**

| Name of formula | Time | | | |
|---|---|---|---|---|
| | Day 0 | Week 1 | Week 2 | Week 4 |
| Formula 1 | 100.4 | N/A | N/A | N/A |
| Formula 2 | 101.7 | 101.5 | 98.3 | 102.3 |
| Formula 3 | 101.2 | 101.5 | 103.4 | 106.0 |
| Formula 4 | 100.9 | 103.6 | 106.8 | 102.8 |
| Formula 5 | 102.6 | 103.4 | 107.4 | 108.5 |

| | | | | |
|---|---|---|---|---|
| Note: N/A indicates that no detection is performed for the sample as its appearance is not up to standard. | | | | |

### (3) Purity

Purity (SEC-HPLC): the results of 4-week observation at 40±2 °C are shown in Table 17, and the trend of change in purity is shown in FIG. 5. The results show that the purity of the formula 2, formula 3, formula 4 and formula 5 samples decreased by 2.6%, 3.0%, 0.7% and 0.7%, respectively, compared to that on day 0 after investigation at 40±2 °C for 4 weeks.

**Table 17. Purity results in stabilizer screening test (SEC-HPLC, %)**

| Sample name | Time | | | |
|---|---|---|---|---|
| | Day 0 | Week 1 | Week 2 | Week 4 |
| Formula 1 | 98.3 | N/A | N/A | N/A |
| Formula 2 | 98.1 | 96.8 | 96.4 | 95.5 |
| Formula 3 | 98.0 | 96.7 | 96.0 | 95.0 |
| Formula 4 | 98.4 | 98.0 | 97.9 | 97.7 |
| Formula 5 | 98.1 | 97.8 | 97.7 | 97.4 |

| | | | | |
|---|---|---|---|---|
| Note: N/A indicates that no detection is performed for the sample as its appearance is not up to standard. | | | | |

Purity (non-reduced CE-SDS): the results of 4-week observation at 40±2 °C are shown in Table 18, and the trend of change in purity is shown in FIG. 6. The results show that the purity of the all formula samples decreased after investigation at 40±2 °C for 4 weeks, and the purity of the formula 2, formula 3, formula 4 and formula 5 samples decreased by 6.8%, 7.2%, 9.1% and 9.2%, respectively, compared to that on day 0.

**Table 18. Purity results in stabilizer screening test (non-reduced CE-SDS, %)**

| Sample name | Time | | | |
|---|---|---|---|---|
| | Day 0 | Week 1 | Week 2 | Week 4 |
| Formula 1 | 98.2 | N/A | N/A | N/A |
| Formula 2 | 98.3 | 94.0 | 94.0 | 91.5 |
| Formula 3 | 98.3 | 93.7 | 93.5 | 91.1 |
| Formula 4 | 97.9 | 95.2 | 92.2 | 88.8 |
| Formula 5 | 98.2 | 93.8 | 92.9 | 89.0 |

| | | | | |
|---|---|---|---|---|
| Note: N/A indicates that no detection is performed for the sample as its appearance is not up to standard | | | | |

### (4) Charge variants (iCIEF)

The results of charge variants after 4-week observation at 40±2 °C are shown in Table 19, and the trend of change in principal component of charge variants is shown in FIG. 7.

The result shows that after investigation at 40±2 °C for 4 weeks, the principal component and the acidic component of the charge variants of all formula samples changed significantly; the principal component decreased, and the acidic component increased, and the trends of change of the samples were basically the same.

**Table 19. Charge variant results in stabilizer screening test (iCIEF, %)**

| Sample name | | Time | | | |
|---|---|---|---|---|---|
| | | Day 0 | Week 1 | Week 2 | Week 4 |
| Formula 1 | Acidic component | 33.3 | N/A | N/A | N/A |
| | Princi pal component | 65.7 | N/A | N/A | N/A |
| | Basic component | 1.0 | N/A | N/A | N/A |
| Formula 2 | Acidic component | 34.3 | 39.8 | 45.6 | 56.8 |
| | Princi pal component | 64.5 | 58.9 | 53.0 | 41.8 |
| | Basic component | 1.1 | 1.3 | 1.4 | 1.4 |
| Formula 3 | Acidic component | 33.8 | 40.7 | 47.3 | 58.3 |
| | Princi pal component | 65.1 | 58.1 | 51.6 | 40.4 |
| | Basic component | 1.1 | 1.2 | 1.2 | 1.3 |
| Formula 4 | Acidic component | 33.0 | 37.7 | 42.9 | 53.5 |
| | Princi pal component | 66.0 | 60.9 | 55.6 | 44.9 |
| | Basic component | 0.9 | 1.5 | 1.6 | 1.5 |
| Formula 5 | Acidic component | 33.7 | 38.2 | 43.5 | 54.1 |
| | Princi pal component | 65.3 | 60.4 | 54.9 | 44.5 |
| | Basic component | 1.0 | 1.4 | 1.6 | 1.4 |

| | | | | | |
|---|---|---|---|---|---|
| Note: N/A indicates that no detection is performed for the sample as its appearance is not up to standard | | | | | |

The results of the stabilizer screening test show that after storage at 40±2 °C for 4 weeks, the formula 2, formula 3, formula 4 and formula 5 samples are up to standard in terms of the appearance and visible particles, the protein content is unchanged, and the purity of the samples is slightly reduced, wherein the formula 4 and the formula 5 samples show significant advantages in purity (SEC-HPLC) and charge variants (iCIEF).

### 4.2. Osmotic pressure test

### 4.2.1 Test procedures

Osmotic pressure of formula 4 and formula 5 samples at 0 h (T0) was measured using a multi-channel osmometer. Each sample was measured twice, and the average of two measurements was taken.

### 4.2.2. Test results

The results of osmotic pressure of formula 4 and formula 5 samples at 0 h (T0) are shown in Table 20.

**Table 20. Measurement results of osmotic pressure**

| Sample name | Osmotic pressure (mOsm/kg) | | |
|---|---|---|---|
| | 1 | 2 | Average |
| Formula 4 | 386 | 383 | 385 |
| Formula 5 | 429 | 418 | 424 |

The osmotic pressure of formula 4 and formula 5 samples is within acceptable ranges of the osmotic pressure of pharmaceutical formulations since the osmotic pressure of human plasma is about 285-310 mOsmol/kg. In addition, the formulation of formula 4 is preferred in view of the fact that the osmotic pressure of formula 4 sample is closer to that of human plasma.

### Example 5: Effect of Metal Chelating Agent on Stability of Formulation

EDTA is a representative metal chelating agent. This example studies the effect of EDTA on the stability of an anti-CD47/PD-L1 bispecific antibody (Kh2NF-PC) protein.

### 5.1. Investigation scheme for effect of EDTA on stability of formulation

A total of 2 formulas are designed, wherein formula 6 is a control group without EDTA, and formula 7 is a group with EDTA at a final concentration of 0.02 mg/mL. The detailed formula information is shown in Table 21. Buffers of the formulas were prepared according to Table 21, and the anti-CD47/PD-L1 bispecific antibody (Kh2NF-PC) protein was exchanged into respective formula solution by ultrafiltration. After the exchange, the protein content of each formula solution was adjusted to about 100.0 mg/mL.

**Table 21. Formula information for effect of EDTA on stability of formulation**

| No. | Formula information |
|---|---|
| Formula 6 | 2.52 mg/mL histidine, 0.79 mg/mL histidine hydrochloride, 37.92 mg/mL arginine hydrochloride, 0.50 mg/mL polysorbate-80, pH 6.5 |
| Formula 7 | 2.52 mg/mL histidine, 0.79 mg/mL histidine hydrochloride, 37.92 mg/mL arginine hydrochloride, 0.50 mg/mL polysorbate-80, 0.02 mg/mL EDTA, pH 6.5 |

The detailed experimental conditions and sampling schedule for the effect of EDTA on stability of formulation are shown in Table 22.

**Table 22. Investigation scheme for effect of EDTA on stability of formulation**

| Experimental conditions | Sampling time points | | | | Test items |
|---|---|---|---|---|---|
| | Day 0 | Week 1 | Week 2 | Week 4 | |
| 40±2 °C | x | x | x | x | Charge variants (CEX-HPLC) and polysorbate-80 |
| | | x | x | x | |

| | | | | | |
|---|---|---|---|---|---|
| Notes: (1) x represents that sampling is performed at this time point. (2) After sampling at the above time points, the obtained samples were first put into an ultra-low temperature refrigerator and frozen for later detection, and then thawed for detection as required. | | | | | |

### 5.2. Experimental results

The results of observation at 40±2 °C until week 4 are shown in Table 23.

**Table 23. Experimental results of EDTA on stability of formulation**

| Sample name | | | Time | | | |
|---|---|---|---|---|---|---|
| | | | Day 0 | Week 1 | Week 2 | Week 4 |
| Formula 6 | Charge variants (CEX-HPLC) | Acidic component | 21.5 | 25.4 | 30.6 | 37.3 |
| | | Princi pal component | 73.6 | 67.8 | 62.0 | 54.0 |
| | | Basic component | 5.0 | 6.8 | 7.4 | 8.7 |
| | Polysorbate-80 (FLD-HPLC) | | 0.49 | 0.18 | 0.10 | 0.11 |
| Formula 7 | Charge variants (CEX-HPLC) | Acidic component | 21.2 | 23.9 | 27.6 | 34.9 |
| | | Princi pal component | 73.9 | 69.8 | 65.7 | 56.9 |
| | | Basic component | 4.9 | 6.2 | 6.6 | 8.2 |
| | Polysorbate-80 (FLD-HPLC) | | 0.48 | 0.48 | 0.48 | 0.48 |

The results in Table 23 show that: according to the charge variant detection results (CEX-HPLC), the main peak of charge variants of formula 7 sample exhibits greater advantage than that of formula 6 sample; according to the detection results of polysorbate-80 (FLD-HPLC), the polysorbate-80 content in the formula 6 sample decreases over time, and the formula 7 sample is superior to the formula 6 sample in that the metal chelating agent EDTA is added to the formula 7 sample and thus degradation of polysorbate-80 due to metal ions is inhibited. EDTA, as an example of a metal chelating agent, is capable of binding to metal ions, and thus can inhibit the degradation of polysorbate-80 in at least two circumstances below. One is that in the whole production process of proteins, including cell culture, purification and other related operation steps, some metal ions may be introduced, leading to oxidative degradation of polysorbate-80 in the presence of both oxygen and metal ions; the other is that some host cell proteins may remain in the proteins in a formula sample, related enzymes capable of degrading polysorbate-80 may exist in these impurity proteins, and the enzymes need metal ions as cofactors to play a catalytic function; therefore, the metal chelating agent added in the formula sample can inhibit the degradation of polysorbate-80 by binding to the metal ions, thereby improving the stability of the formula.

Thus, the most preferred formulation scheme is determined to be: about 100.0 mg/mL recombinant anti-cluster of differentiation 47 (CD47) and anti-programmed death ligand 1 (PD-L1) bispecific antibody, about 2.52 mg/mL histidine, 0.79 mg/mL histidine hydrochloride, 37.92 mg/mL arginine hydrochloride, 0.50 mg/ml polysorbate-80, 0.02 mg/mL EDTA, pH 6.5.

### Example 6. Investigation on Stability of 500 L Preparation

A 500 L preparation was prepared using the formulation scheme of Example 5 (about 100.0 mg/mL recombinant anti-cluster of differentiation 47 (CD47) and anti-programmed death ligand 1 (PD-L1) bispecific antibody, about 2.52 mg/mL histidine, 0.79 mg/mL histidine hydrochloride, 37.92 mg/mL arginine hydrochloride, 0.50 mg/mL polysorbate-80, 0.02 mg/mL EDTA, pH 6.5) for stability investigation.

### 6.1. Preparation for stability investigation and investigation scheme

500 L of the following preparation was prepared for stability investigation: 101.8 mg/mL recombinant anti-CD47/PD-L1 bispecific antibody protein, 2.52 mg/mL histidine, 0.79 mg/mL histidine hydrochloride, 37.92 mg/mL arginine hydrochloride, 0.50 mg/mL polysorbate-80, 0.02 mg/mL EDTA, pH 6.5.

**Table 24. Investigation scheme for stability of preparation**

| Research items | Investigation conditions | Investigation time points |
|---|---|---|
| Long-term stability study | 5±3 °C | Month 0, 3, 6, 9, 12 , 18 , 24 |
| Accelerated stability study | 25±2 °C/60±5% relative humidity (RH) | Month 0, 1, 2, 3, 4, 5, 6 |
| Forced condition test | 40±2 °C/75±5% RH | Week 0, 2, 4, 8 |

**Table 25. Quality criteria**

| Test items | | Method | Quality criteria |
|---|---|---|---|
| 1. Isoelectric point | | iCIEF | 8.6-9.2 |
| 2. Purity and impurities | Purity | SEC-HPLC (%) | Main peak: should be ≥ 95.0%² |
| | | | Polymer: reported data¹ (%) |
| | | | Fragment: reported data (%) |
| | | Non-reduced CE-SDS (%) | Main peak: should be ≥ 90.0%³ Fragment: reported data (%) |
| | | Reduced CE-SDS (%) | Heavy and light chain content: should be ≥ 90.0%⁴ |
| | | | Non-glycosylated heavy chain: reported data (%) |
| | | | Fragment: reported data (%) |
| | Charge variants | CEX-HPLC (%) | Principal component: should be ≥ 44.9%⁵ |
| | | | Acidic component: reported data (%) |
| | | | Basic component: reported data (%) |
| 3. Potency | Biological activity of anti-CD47 end | Cell competitive ELISA (%) | Should be 70-130% |
| | Biological activity of anti-PD-L1 end | Luciferase reporter gene cell assay (%) | Should be 70-130% |
| 4. Protein content | | UV gravimetric method (mg/mL) | Should be 90.0-110.0 mg/mL |
| 5. Others | Appearance | Observation | Should be clear to slightly opalescent, colorless to pale yellow liquid, no particles |
| | pH value | Method for determining pH | Should be 6.2-6.8 |
| | Osmotic pressure | Osmolarity assay | Should be 295-395 mOsmol/kg |
| | Loading capacity | Gravimetric method | Should be no less than the labeled amount 1.0 mL/piece (usually 1.06-1.13 mL/piece, the same applies hereinafter) |
| | Insoluble microparticles | Light blockage | The number of microparticles with the diameter ≥ 10 µm in each piece should be ≤ 6000 (calculation according to 1.0 mL/piece; the same applies hereinafter) |
| | | | The number of microparticles with the diameter ≥ 25 µm in each piece should be ≤ 600 |
| | Visible particles | Test for visible particles | Should comply with specification⁶ |
| | Sterile | Membrane filtration | Should be no growing bacteria |
| | Bacterial endotoxins^{∗} | Dynamic chromogenic method^{∗} | Should be ≤ 2.00 EU/mL |
| | Examination of abnormal toxicity | Mouse method | Should comply with specification |
| | Polysorbate-80 content | FLD-HPLC | Should be 0.3-0.7 mg/mL |

| | | | |
|---|---|---|---|
| Notes: 1. The reported data refers to that the acceptance range is not set for the detection item, and the data actually detected can be directly reported. The same applies hereinafter. | | | |

2. In the SEC-HPLC, the main peak + polymer + fragment is 100%, and as long as the main peak is ≥ 95%, the remaining 5% or less is the amount of the fragment and the polymer, and thus the data of the amount of the fragment and the polymer is reported data. The same applies hereinafter.

3. In the non-reduced CE-SDS, the main peak + fragment is 100%, and as long as the main peak is ≥ 90%, the remaining 10% or less is the amount of the fragment, and thus the data of the amount of the fragment is reported data. The same applies hereinafter.

4. In the reduced CE-SDS, the heavy and light chain content + the non-glycosylated heavy chain + the fragment is 100%, and as long as the heavy and light chain content is ≥ 90%, the remaining 10% or less is the amount of the non-glycosylated heavy chain and the fragment, and thus the data of both the amount of the non-glycosylated heavy chain and the amount of the fragment is reported data. The same applies hereinafter.

5. In the CEX-HPLC, the principal component + acidic component + basic component is 100%, and as long as the amount of the principal component is ≥ 44.9%, the remaining 55.1% or less is the amount of the acidic component and the basic component, and thus the data of both the amount of the acidic component and the amount of the basic component are reported data. The same applies hereinafter.

6. The specification is the relevant specification in the Pharmacopoeia of the People's Republic of China (2015 edition, volume III), and the same applies hereinafter; For example, General Rules 0904 "Test for Visible Particles" stipulates the inspection of visible particles.

### 6.2. Results of stability investigation

The results of stability investigation for the 500 L preparation are shown in the table below.

**Table 26. Results of long-term stability study (5±3 °C)**

| Test items | Acceptance criteria | Month 0 | Month 1 | Month 3 | Month 6 |
|---|---|---|---|---|---|
| Isoelectric point (iCIEF) | Main peak pI: 8.6-9.2 | Main peak pI: 9.0 | N/A | N/A | N/A |
| Charge variants (CEX-HPLC) (%) | Principal component: should be ≥ 44.9% | 73.1 | 72.5 | 72.0 | 98.8 |
| | Acidic component: reported data | 22.4 | 23.0 | 23.5 | 0.7 |
| | Basic component: reported data | 4.5 | 4.4 | 4.5 | 0.5 |
| Purity (SEC-HPLC, %) | Main peak: should be ≥ 95.0% | 99.3 | 99.3 | 99.2 | 97.7 |
| | Polymer: reported data | 0.4 | 0.4 | 0.5 | 2.3 |
| | Fragment: reported data | 0.3 | 0.3 | 0.3 | N/A |
| Purity (Non-reduced CE-SDS) (%) | Main peak: should be ≥ 90.0% | 97.9 | 97.7 | 97.6 | N/A |
| | Fragment: reported data | 2.1 | 2.3 | 2.4 | N/A |
| Purity (Reduced CE-SDS) (%) | Main peak: ≥ 90.0% | 99.0 | N/A | N/A | 71.0 |
| | NGHC_{CD47}: reported data | 0.2 | N/A | N/A | 23.7 |
| | Fragment: reported data | 0.8 | N/A | N/A | 5.3 |
| Biological activity of anti-CD47 end (Cell competitive ELISA) (%) | Should be 70-130% | 93 | 99 | 89 | 101 |
| Biological activity of anti-PD-L1 end (Luciferase reporter gene cell assay) (%) | Should be 70-130% | 102 | 94 | 116 | 97 |
| Protein content (UV method, mg/mL) | Should be 90.0-110.0 mg/mL | 101.8 | 101.3 | 102.2 | 101.6 |
| Appearance (Observation) | Should be clear to slightly opalescent, colorless to pale yellow liquid, no particles | Clear and pale yellow liquid, no particles | Clear and pale yellow liquid, no particles | Clear and colorless liquid, no particles | Clear and colorless liquid, no particles |
| Visible particles (Test for visible particles) | Should comply with specification | Comply with specification | N/A | N/A | N/A |
| pH value (Method for determining pH) | Should be 6.2-6.8 | 6.6 | 6.6 | 6.6 | 6.6 |
| Insoluble microparticles (Light blockage) | The number of microparticles with the diameter ≥ 10 µm in each piece should be ≤ 6000 | 10 | N/A | N/A | N/A |
| | The number of microparticles with the diameter ≥ 25 µm in each piece should be ≤ 600 | 1 | N/A | N/A | N/A |
| Loading (Gravimetric method) | Should not be lower than its labeled amount (1.0 mL/piece) | Comply with specification | N/A | N/A | N/A |
| Osmotic pressure (Osmolarity assay) (mOsmol/kg) | Should be 295-395 mOsmol/kg | 340 | N/A | N/A | N/A |
| Polysorbate-80 content (HPLC-FLD) (mg/mL) | Should be 0.3-0.7 mg/mL | 0.50 | N/A | N/A | N/A |
| Sterile (Membrane filtration) | Should be no growing bacteria | Comply with specification | N/A | N/A | N/A |
| Bacterial endotoxins (Dynamic chromogenic method) (EU/mL) | Should be ≤ 2.00 EU/mL | < 0.40 | N/A | N/A | N/A |
| Container seal integrity (Staining method) | The absorbance at 647 nm should be ≤ 0.008 | Comply with specification | N/A | N/A | N/A |
| Abnormal toxicity (Examination of abnormal toxicity) | Should comply with specification | Comply with specification | N/A | N/A | N/A |

| | | | | | |
|---|---|---|---|---|---|
| Notes: 1. N/A represents that no detection is performed, and the corresponding index is generally only detected for two endpoints, and if the two endpoints meet the requirements, the value of each point therebetween is considered to meet the requirements. | | | | | |

2. In the reduced CE-SDS, the main peak + NGHC_{CD47} + fragment is 100%, and as long as the main peak is ≥ 90%, the remaining 10% or less is the amount of NGHC_{CD47} chain and fragment, and thus the data of the amount of NGHC_{CD47} and the amount of fragment is reported data.

As can be seen from the detection results in Table 26, the preparation met the quality criteria after 6 months.

**Table 27. Results of accelerated stability study (25±2 °C/60±5% RH)**

| Test items | Quality criteria | Investigation time | | | | |
|---|---|---|---|---|---|---|
| | | Month 0 | Month 1 | Month 2 | Month 3 | Month 6 |
| Charge variants (CEX-HPLC) (%) | Princi pal component: should be ≥ 44.9% | 73.1 | 69.2 | 66.5 | 62.8 | 97.8 |
| | Acidic component: reported data | 22.4 | 25.4 | 27.5 | 30.9 | 1.2 |
| | Basic component: reported data | 4.5 | 5.4 | 6.1 | 6.3 | 1.0 |
| Purity (SEC-HPLC, %) | Main peak: should be ≥ 95.0% | 99.3 | 98.9 | 98.7 | 98.6 | 93.5 |
| | Polymer: reported data | 0.4 | 0.7 | 0.8 | 0.9 | 6.5 |
| | Fragment: reported data | 0.3 | 0.4 | 0.5 | 0.5 | 54.6 |
| Purity (Non-reduced CE-SDS) (%) | Main peak: should be ≥ 90.0% | 97.9 | 97.2 | 96.5 | 95.8 | 37.3 |
| | Fragment: reported data | 2.1 | 2.8 | 3.5 | 4.2 | 8.1 |
| Biological activity of anti-CD47 end (Cell competitive ELISA) (%) | Should be 70-130% | 93 | 97 | 81 | 116 | 90 |
| Biological activity of anti-PD-L1 end (Luciferase reporter gene cell assay) (%) | Should be 70-130% | 102 | 111 | 91 | 102 | 89 |
| Protein content (UV method, mg/mL) | Should be 90.0-110.0 mg/mL | 101.8 | 101.4 | 103.5 | 102.3 | 101.3 |
| Appearance (Observation) | Should be clear to slightly opalescent, colorless to pale yellow liquid, no particles | Clear and pale yellow liquid, no particles | Clear and pale yellow liquid, no particles | Clear and colorless liquid, no particles | Clear and colorless liquid, no particles | Clear and colorless liquid, no particles |
| Visible particles (Test for visible particles) | Should comply with specification | Comply with specification | N/A | N/A | N/A | Comply with specification |
| pH value (Method for determining pH) | Should be 6.2-6.8 | 6.6 | 6.6 | 6.6 | 6.6 | 6.6 |
| Insoluble microparticles (Light blockage) | The number of microparticles with the diameter ≥ 10 µm in each piece should be ≤ 6000 | 10 | N/A | N/A | N/A | 8 |
| | The number of microparticles with the diameter ≥ 25 µm in each piece should be ≤ 600 | 1 | N/A | N/A | N/A | 1 |
| Polysorbate-8 0 content (HPLC- FLD) (mg/mL) | Should be 0.3-0.7 mg/mL | 0.50 | N/A | N/A | N/A | 0.5 |
| Note: N/A represents that no detection is performed, and the corresponding index is generally only detected for two endpoints, and if the two endpoints meet the requirements, the value of each point therebetween is considered to meet the requirements. | | | | | | |

As can be seen from the detection results in Table 27, in the accelerated stability test, the preparation is up to standard for all the indexes after 6 months.

**Table 28. Results of forced condition test (40±2 °C/75±5% RH)**

| Test items | Acceptance criteria | Investigation time | | | |
|---|---|---|---|---|---|
| | | Month 0 | Week 2 | Week 4 | Week 8 |
| Charge variants (CEX-HPLC) (%) | Princi pal component: should be ≥ 44.9% | 73.1 | 63.1 | 54.4 | 41.0^{a} |
| | Acidic component: reported data | 22.4 | 29.2 | 37.4 | 48.9 |
| | Basic component: reported data | 4.5 | 7.7 | 8.3 | 10.0 |
| Purity (SEC-HPLC, %) | Main peak: should be ≥ 95.0% | 99.3 | 98.7 | 98.2 | 97.0 |
| | Polymer: reported data | 0.4 | 1.0 | 1.2 | 1.8 |
| | Fragment: reported data | 0.3 | 0.2 | 0.5 | 1.2 |
| Purity (Non-reduced CE-SDS) (%) | Main peak: should be ≥ 90.0% | 97.9 | 95.4 | 93.4 | 89.3^{b} |
| | Fragment: reported data | 2.1 | 4.6 | 6.6 | 10.7 |
| Biological activity of anti-CD47 end (Cell competitive ELISA) (%) | Should be 70-130% | 93 | 95 | 99 | 89 |
| Biological activity of anti-PD-L1 end (Luciferase reporter gene cell assay) (%) | Should be 70-130% | 102 | 100 | 97 | 93 |
| Protein content (UV method, mg/mL) | Should be 90.0-110.0 mg/mL | 101.8 | 102.4 | 101.5 | 102.7 |
| Appearance (Observation) | Should be clear to slightly opalescent, colorless to pale yellow liquid, no particles | Clear and pale yellow liquid, no particles | Clear and pale yellow liquid, no particles | Clear and colorless liquid, no particles | Clear and pale yellow liquid, no particles |
| Visible particles (Test for visible particles) | Should comply with specification | Comply with specification | N/A | N/A | Comply with specification |
| pH value (Method for determining pH) | Should be 6.2-6.8 | 6.6 | 6.6 | 6.6 | 6.6 |
| Insoluble microparticles (Light blockage) | The number of microparticles with the diameter ≥ 10 µm in each piece should be ≤ 6000 | 10 | N/A | N/A | 7 |

| Test items | Acceptance criteria | Investigation time | | | |
|---|---|---|---|---|---|
| | | Month 0 | Week 2 | Week 4 | Week 8 |
| | The number of microparticles with the diameter ≥ 25 µm in each piece should be ≤ 600 | 1 | N/A | N/A | 1 |
| Polysorbate-80 content (HPLC-FLD) (mg/mL) | Should be 0.3-0.7 mg/mL | 0.5 | N/A | N/A | 0.5 |

| | | | | | |
|---|---|---|---|---|---|
| Note: N/A represents that no detection is performed, and the corresponding index is generally only detected for two endpoints, and if the two endpoints meet the requirements, the value of each point therebetween is considered to meet the requirements. | | | | | |

As can be seen from the detection results in Table 28, in the forced condition stability test, the preparation is up to standard for all the indexes after 8 weeks.

In conclusion, it is found through the above experiments that the formulation scheme disclosed herein satisfies the stability requirement for formulations in the scale-up production.

The exemplary embodiments of the present invention have been described above. It should be understood by those skilled in the art that these contents are merely exemplary, and various other replacements, adaptations and modifications can be made within the scope of the present invention. Accordingly, the present invention is not limited to the specific embodiments listed herein.

## Claims

1. A liquid antibody formulation, comprising:
(i) an anti-CD47/PD-L1 bispecific antibody protein;
(ii) a buffer;
(iii) a stabilizer;
(iv) a surfactant; and
optionally, (v) a metal chelating agent (e.g., EDTA), wherein
the anti-CD47/PD-L1 bispecific antibody protein is a triple-chain antibody, wherein the triple-chain antibody comprises a VH/VL pair specifically binding to CD47 on a first polypeptide chain and a second polypeptide chain as a first antigen-binding site, and a first VHH and a second VHH specifically binding to PD-L1 on a third polypeptide chain as a second single domain antigen-binding site and a third single domain antigen-binding site, respectively; or comprises a VH/VL pair specifically binding to PD-L1 on a first polypeptide chain and a second polypeptide chain as a first antigen-binding site, and a first VHH and a second VHH specifically binding to CD47 on a third polypeptide chain as a second single domain antigen-binding site and a third single domain antigen-binding site, respectively;
preferably, the pH of the liquid antibody formulation is about 6.4-7.0, e.g., about 6.4, 6.5, 6.8 or 7.0.

2. The liquid antibody formulation according to claim 1, wherein the concentration of the anti-CD47/PD-L1 bispecific antibody protein in the liquid antibody formulation is about 1-200 mg/mL, preferably about 5-150 mg/mL, e.g., about 5, 10, 20, 30, 40, 50, 60, 70, 80, 90, 100, 110, 120, 130, 140 or 150 mg/mL.

3. The liquid antibody formulation according to claim 1 or 2, wherein the buffer in the liquid antibody formulation is selected from histidine, histidine hydrochloride and a combination thereof; the concentration of the buffer is preferably about 1-30 mM, and more preferably about 5-25 mM, e.g. about 5, 10, 15, 20 or 25 mM.

4. The liquid antibody formulation according to any one of claims 1-3, wherein the stabilizer is selected from sorbitol, sucrose, trehalose, arginine, arginine hydrochloride and any combination thereof, and is preferably sucrose, arginine and/or arginine hydrochloride; the concentration of the stabilizer is preferably about 50-500 mM, and more preferably about 100-400 mM, e.g., about 100, 150, 200, 250, 300, 350 or 400 mM.

5. The liquid antibody formulation according to any one of claims 1-4, wherein the liquid antibody formulation comprises arginine hydrochloride as the stabilizer; preferably, arginine hydrochloride is present in an amount of about 50-250 mM, preferably about 100-200 mM (e.g., about 100, 110, 120, 130, 140, 150, 160, 170, 180, 190 or 200 mM); and/or the liquid antibody formulation comprises sucrose as the stabilizer; preferably, sucrose is present in an amount of about 50-250 mM, preferably about 100-200 mM (e.g., about 100, 110, 120, 130, 140, 150, 160, 170, 180, 190 or 200 mM).

6. The liquid antibody formulation according to any one of claims 1-5, wherein the surfactant in the liquid antibody formulation is selected from polysorbate surfactants, and is preferably polysorbate-80.

7. The liquid antibody formulation according to any one of claims 1-6, wherein the concentration of the surfactant is about 0.1-1 mg/mL, preferably about 0.2-0.8 mg/mL, e.g., about 0.2, 0.3, 0.4, 0.5, 0.6, 0.7 or 0.8 mg/mL.

8. The liquid antibody formulation according to any one of claims 1-7, wherein the liquid formulation further comprises a metal chelating agent (e.g., EDTA), such as about 0.002-0.2 mg/mL metal chelating agent (e.g., EDTA), preferably about 0.01-0.1 mg/mL, e.g., about 0.01, 0.02, 0.03, 0.04, 0.05, 0.06, 0.08 or 0.1 mg/mL, metal chelating agent (e.g., EDTA).

9. The liquid antibody formulation according to claim 1, wherein the VH/VL pair specifically binding to CD47 on the first polypeptide chain and the second polypeptide chain of the anti-CD47/PD-L1 bispecific antibody protein, as the first antigen-binding site, comprises a VH CDR1 set forth in GSIEHYYWS (SEQ ID NO: 3), a VH CDR2 set forth in YIYYSGSTNYNPSLKS (SEQ ID NO: 4), a VH CDR3 set forth in ARGKTGSAA (SEQ ID NO: 5), a VL CDR1 set forth in RASQGISRWLA (SEQ ID NO: 10), a VL CDR2 set forth in AASSLQS (SEQ ID NO: 11) and a VL CDR3 set forth in QQTVSFPIT (SEQ ID NO: 12) derived from anti-CD47 antibody ADI-29341, or sequences having one, two, three, four, five, six or more amino acid changes (e.g., amino acid substitutions or deletions) compared with one or more CDRs of the 6 CDRs; the second single domain antigen-binding site and the third single domain antigen-binding site specifically binding to PD-L1 on the third polypeptide chain both comprise a CDR1 set forth in AYTISRNSMG (SEQ ID NO: 17), a CDR2 set forth in IESDGST (SEQ ID NO: 18) and a CDR3 set forth in AAPKVGLGPRTALGHLAFMTLPALNY (SEQ ID NO: 19), or sequences having one, two, three, four, five, six or more amino acid changes (e.g., amino acid substitutions or deletions) compared with one or more CDRs of the 3 CDRs;
more preferably, the VH/VL pair specifically binding to CD47 on the first polypeptide chain and the second polypeptide chain, as the first antigen-binding site, comprises paired heavy chain variable region/light chain variable region sequences of SEQ ID NOs: 2/9 derived from anti-CD47 antibody ADI-29341, or sequences having at least 90%, 91%, 92%, 93%, 94%, 95%, 96%, 97%, 98%, 99% or higher sequence identity to the paired heavy chain variable region/light chain variable region sequences, and the second single domain antigen-binding site and the third single domain antigen-binding site specifically binding to PD-L1 on the third polypeptide chain both comprise sequences set forth in SEQ ID NO: 15 and/or SEQ ID NO: 16, or sequences substantially identical (e.g., having at least 80%, 85%, 90%, 92%, 95%, 97%, 98%, 99% or higher identity) thereto;
most preferably, the triple-chain antibody comprises a first polypeptide chain set forth in SEQ ID NO: 1, a second polypeptide chain set forth in SEQ ID NO: 8, and a third polypeptide chain set forth in SEQ ID NO: 14 or SEQ ID NO: 22, or sequences substantially identical (e.g., having at least 80%, 85%, 90%, 92%, 95%, 97%, 98%, 99% or higher identity) to any one of the sequences.

10. The liquid antibody formulation according to any one of claims 1-9, wherein the anti-CD47/PD-L1 bispecific antibody protein is recombinantly expressed in HEK293 cells or CHO cells.

11. The liquid antibody formulation according to any one of claims 1-10, wherein the liquid formulation is an injection, preferably for subcutaneous or intravenous injection, or an infusion, e.g., for intravenous infusion.

12. The liquid antibody formulation according to any one of claims 1-11, wherein the liquid antibody formulation comprises:
(i) about 1-200 mg/mL anti-CD47/PD-L1 bispecific antibody protein;
(ii) about 1-30 mM histidine and/or histidine hydrochloride;
(iii) about 50-500 mM sucrose, arginine and/or arginine hydrochloride; and
(iv) about 0.1-1 mg/mL polysorbate-80, wherein
optionally, the liquid formulation further comprises 0.002-0.2 mg/mL metal chelating agent (e.g., EDTA), wherein
the pH of the liquid formulation is about 6.4-7.0, preferably about 6.5;
for example, the liquid antibody formulation comprises:
(i) about 50-150 mg/mL anti-CD47/PD-L1 bispecific antibody protein;
(ii) about 3-25 mM histidine and/or histidine hydrochloride;
(iii) about 150-300 mM sucrose, arginine and/or arginine hydrochloride; and
(iv) about 0.2-0.8 mg/mL polysorbate-80, wherein
optionally, the liquid formulation further comprises about 0.01-0.1 mg/mL metal chelating agent (e.g., EDTA), wherein
the pH of the liquid formulation is about 6.4-7.0, preferably about 6.5; or
the liquid antibody formulation comprises:
(i) about 100 mg/mL anti-CD47/PD-L1 bispecific antibody protein;
(ii) about 20 mM histidine;
(iii) about 180 mM arginine hydrochloride; and
(iv) about 0.2 mg/mL polysorbate-80, wherein
optionally, the liquid formulation further comprises a metal chelating agent (e.g., EDTA), e.g., about 0.02 mg/mL EDTA, wherein
the pH of the liquid formulation is about 6.4-7.0, preferably about 6.5; or
the liquid antibody formulation comprises:
(i) about 100 mg/mL anti-CD47/PD-L1 bispecific antibody protein;
(ii) about 20 mM histidine;
(iii) about 100 mM arginine hydrochloride and 4% sucrose; and
(iv) about 0.2 mg/mL polysorbate-80, wherein
optionally, the liquid formulation further comprises a metal chelating agent (e.g., EDTA), e.g., about 0.02 mg/mL EDTA, wherein
the pH of the liquid formulation is about 6.4-7.0, preferably about 6.5; or
the liquid antibody formulation comprises:
(i) about 100 mg/mL anti-CD47/PD-L1 bispecific antibody protein;
(ii) about 2.52 mg/mL histidine and about 0.79 mg/mL histidine hydrochloride;
(iii) about 37.92 mg/mL arginine hydrochloride; and
(iv) about 0.5 mg/mL polysorbate-80, wherein
optionally, the liquid formulation further comprises a metal chelating agent (e.g., EDTA), e.g., about 0.02 mg/mL EDTA, wherein
the pH of the liquid formulation is about 6.4-7.0, preferably about 6.5.

13. The liquid antibody formulation according to any one of claims 1-12, wherein the formulation is stable after storage, e.g., at 2-8 °C for at least 24 months, at room temperature for at least 3 months, or at 40±2 °C for 1 month, and preferably has one or more of the following characteristics:
(i) a purity greater than 90%, preferably greater than 95%, 96%, 97%, 98% or 99%, as measured by SEC-HPLC;
(ii) a purity greater than 85%, preferably greater than 86%, 87%, 88%, 89%, 90%, 91% or 92%, as measured by reduced or non-reduced CE-SDS;
(iii) total change ≤ 50% in components (principal component, acidic component and basic component) of the anti-CD47/PD-L1 bispecific antibody protein in the formulation, e.g., ≤ 48%, 46%, 44%, 42% or 40%, relative to an initial value on day 0 of storage, as measured by iCIEF;
(iv) total change ≤ 40% in components (principal component, acidic component and basic component) of the anti-CD47/PD-L1 bispecific antibody protein in the formulation, e.g., ≤ 38%, 36%, 34%, 32% or 30%, relative to an initial value on day 0 of storage, as measured by CEX-HPLC; and
(v) relative binding activity of the anti-CD47/PD-L1 bispecific antibody protein in the formulation of 70-130%, e.g., 70%, 80%, 90%, 100%, 110%, 120% or 130%, relative to an initial value on day 0 of storage, as measured by ELISA.

14. A solid antibody formulation, obtained by solidifying the liquid antibody formulation according to any one of claims 1-13, wherein, the solid antibody formulation is, e.g., in the form of lyophilized powder for injection.

15. A delivery device, comprising the liquid antibody formulation according to any one of claims 1-13 or the solid antibody formulation according to claim 14.

16. A pre-filled syringe, comprising the liquid antibody formulation according to any one of claims 1-13 or the solid antibody formulation according to claim 14 for use in intravenous or intramuscular injection.

17. Use of the liquid antibody formulation according to any one of claims 1-13 or the solid antibody formulation according to claim 14 in preparing a medicament for treating, preventing or delaying a disorder associated with an SIRPα/CD47 signaling pathway and a PD1/PD-L1 signaling pathway, wherein the disorder includes, e.g., various solid tumors and blood diseases (e.g., leukaemia, lymphoma, or myeloma, e.g. multiple myeloma), autoimmune diseases, acute and chronic inflammatory disorders, infectious diseases and metastatic lesions.


SEQUENCE LISITNG
<110>  Innovent Biologics (Suzhou) Co., Ltd.
<120>  FORMULATION COMPRISING ANTI-CD47/PD-L1 BISPECIFIC ANTIBODY, METHOD
FOR PREPARING SAME AND USE THEREOF
<130>
<160>  22
<170>  PatentIn version 3.3
<210>  1
<211>  444
<212>  PRT
<213>  Artificial Sequence
<220>
<223>  First peptide chain of anti-CD47/PDL1 bispecific antibody
<400>  1
Gln Val Gln Leu Gln Glu Ser Gly Pro Gly Leu Val Lys Pro Ser Glu
1               5                   10                  15
Thr Leu Ser Leu Thr Cys Thr Val Ser Gly Gly Ser Ile Glu His Tyr
            20                  25                  30
Tyr Trp Ser Trp Ile Arg Gln Pro Pro Gly Lys Gly Leu Glu Trp Ile
        35                  40                  45
Gly Tyr Ile Tyr Tyr Ser Gly Ser Thr Asn Tyr Asn Pro Ser Leu Lys
    50                  55                  60
Ser Arg Val Thr Ile Ser Val Asp Thr Ser Lys Asn Gln Phe Ser Leu
65                  70                  75                  80
Lys Leu Ser Ser Val Thr Ala Ala Asp Thr Ala Val Tyr Tyr Cys Ala
                85                  90                  95
Arg Gly Lys Thr Gly Ser Ala Ala Trp Gly Gln Gly Thr Leu Val Thr
            100                 105                 110
Val Ser Ser Ala Ser Thr Lys Gly Pro Ser Val Phe Pro Leu Ala Pro
        115                 120                 125
Ser Ser Lys Ser Thr Ser Gly Gly Thr Ala Ala Leu Gly Cys Leu Val
    130                 135                 140
Lys Asp Tyr Phe Pro Glu Pro Val Thr Val Ser Trp Asn Ser Gly Ala
145                 150                 155                 160
Leu Thr Ser Gly Val His Thr Phe Pro Ala Val Leu Gln Ser Ser Gly
                165                 170                 175
Leu Tyr Ser Leu Ser Ser Val Val Thr Val Pro Ser Ser Ser Leu Gly
            180                 185                 190
Thr Gln Thr Tyr Ile Cys Asn Val Asn His Lys Pro Ser Asn Thr Lys
        195                 200                 205
Val Asp Lys Lys Val Glu Pro Lys Ser Cys Asp Lys Thr His Thr Cys
    210                 215                 220
Pro Pro Cys Pro Ala Pro Glu Ala Ala Gly Gly Pro Ser Val Phe Leu
225                 230                 235                 240
Phe Pro Pro Lys Pro Lys Asp Thr Leu Met Ile Ser Arg Thr Pro Glu
                245                 250                 255
Val Thr Cys Val Val Val Asp Val Ser His Glu Asp Pro Glu Val Lys
            260                 265                 270
Phe Asn Trp Tyr Val Asp Gly Val Glu Val His Asn Ala Lys Thr Lys
        275                 280                 285
Pro Arg Glu Glu Gln Tyr Asn Ser Thr Tyr Arg Val Val Ser Val Leu
    290                 295                 300
Thr Val Leu His Gln Asp Trp Leu Asn Gly Lys Glu Tyr Lys Cys Lys
305                 310                 315                 320
Val Ser Asn Lys Ala Leu Pro Ala Pro Ile Glu Lys Thr Ile Ser Lys
                325                 330                 335
Ala Lys Gly Gln Pro Arg Glu Pro Gln Val Tyr Thr Leu Pro Pro Cys
            340                 345                 350
Arg Asp Glu Leu Thr Lys Asn Gln Val Ser Leu Trp Cys Leu Val Lys
        355                 360                 365
Gly Phe Tyr Pro Ser Asp Ile Ala Val Glu Trp Glu Ser Asn Gly Gln
    370                 375                 380
Pro Glu Asn Asn Tyr Lys Thr Thr Pro Pro Val Leu Asp Ser Asp Gly

385                 390                 395                 400
Ser Phe Phe Leu Tyr Ser Lys Leu Thr Val Asp Lys Ser Arg Trp Gln
                405                 410                 415
Gln Gly Asn Val Phe Ser Cys Ser Val Met His Glu Ala Leu His Asn
            420                 425                 430
His Tyr Thr Gln Lys Ser Leu Ser Leu Ser Pro Gly
        435                 440
<210>  2
<211>  115
<212>  PRT
<213>  Artificial Sequence
<220>
<223>  VH of anti-CD47 antibody ADI-29341
<400>  2
Gln Val Gln Leu Gln Glu Ser Gly Pro Gly Leu Val Lys Pro Ser Glu
1               5                   10                  15
Thr Leu Ser Leu Thr Cys Thr Val Ser Gly Gly Ser Ile Glu His Tyr
            20                  25                  30
Tyr Trp Ser Trp Ile Arg Gln Pro Pro Gly Lys Gly Leu Glu Trp Ile
        35                  40                  45
Gly Tyr Ile Tyr Tyr Ser Gly Ser Thr Asn Tyr Asn Pro Ser Leu Lys
    50                  55                  60
Ser Arg Val Thr Ile Ser Val Asp Thr Ser Lys Asn Gln Phe Ser Leu
65                  70                  75                  80
Lys Leu Ser Ser Val Thr Ala Ala Asp Thr Ala Val Tyr Tyr Cys Ala
                85                  90                  95
Arg Gly Lys Thr Gly Ser Ala Ala Trp Gly Gln Gly Thr Leu Val Thr
            100                 105                 110
Val Ser Ser
        115
<210>  3
<211>  9
<212>  PRT
<213>  Artificial Sequence
<220>
<223>  VH CDR1 of anti-CD47 antibody ADI-29341
<400>  3
Gly Ser Ile Glu His Tyr Tyr Trp Ser
1               5
<210>  4
<211>  16
<212>  PRT
<213>  Artificial Sequence
<220>
<223>  VH CDR2 of anti-CD47 antibody ADI-29341
<400>  4
Tyr Ile Tyr Tyr Ser Gly Ser Thr Asn Tyr Asn Pro Ser Leu Lys Ser
1               5                   10                  15
<210>  5
<211>  9
<212>  PRT
<213>  Artificial Sequence
<220>
<223>  VH CDR3 of anti-CD47 antibody ADI-29341
<400>  5
Ala Arg Gly Lys Thr Gly Ser Ala Ala
1               5
<210>  6
<211>  108
<212>  PRT
<213>  Artificial Sequence
<220>
<223>  CH1 amino acid sequence derived from human IgG1

<400> 6
Ala Ser Thr Lys Gly Pro Ser Val Phe Pro Leu Ala Pro Ser Ser Lys
1               5                   10                  15
Ser Thr Ser Gly Gly Thr Ala Ala Leu Gly Cys Leu Val Lys Asp Tyr
            20                  25                  30
Phe Pro Glu Pro Val Thr Val Ser Trp Asn Ser Gly Ala Leu Thr Ser
        35                  40                  45
Gly Val His Thr Phe Pro Ala Val Leu Gln Ser Ser Gly Leu Tyr Ser
    50                  55                  60
Leu Ser Ser Val Val Thr Val Pro Ser Ser Ser Leu Gly Thr Gln Thr
65                  70                  75                  80
Tyr Ile Cys Asn Val Asn His Lys Pro Ser Asn Thr Lys Val Asp Lys
                85                  90                  95
Lys Val Glu Pro Lys Ser Cys Asp Lys Thr His Thr
            100                 105
<210> 7
<211> 221
<212> PRT
<213> Artificial Sequence
<220>
<223> Fc region amino acid sequence derived from human IgG1
<400> 7
Cys Pro Pro Cys Pro Ala Pro Glu Ala Ala Gly Gly Pro Ser Val Phe
1               5                   10                  15
Leu Phe Pro Pro Lys Pro Lys Asp Thr Leu Met Ile Ser Arg Thr Pro
            20                  25                  30
Glu Val Thr Cys Val Val Val Asp Val Ser His Glu Asp Pro Glu Val
        35                  40                  45
Lys Phe Asn Trp Tyr Val Asp Gly Val Glu Val His Asn Ala Lys Thr
    50                  55                  60
Lys Pro Arg Glu Glu Gln Tyr Asn Ser Thr Tyr Arg Val Val Ser Val
65                  70                  75                  80
Leu Thr Val Leu His Gln Asp Trp Leu Asn Gly Lys Glu Tyr Lys Cys
                85                  90                  95
Lys Val Ser Asn Lys Ala Leu Pro Ala Pro Ile Glu Lys Thr Ile Ser
            100                 105                 110
Lys Ala Lys Gly Gln Pro Arg Glu Pro Gln Val Tyr Thr Leu Pro Pro
        115                 120                 125
Cys Arg Asp Glu Leu Thr Lys Asn Gln Val Ser Leu Trp Cys Leu Val
    130                 135                 140
Lys Gly Phe Tyr Pro Ser Asp Ile Ala Val Glu Trp Glu Ser Asn Gly
145                 150                 155                 160
Gln Pro Glu Asn Asn Tyr Lys Thr Thr Pro Pro Val Leu Asp Ser Asp
                165                 170                 175
Gly Ser Phe Phe Leu Tyr Ser Lys Leu Thr Val Asp Lys Ser Arg Trp
            180                 185                 190
Gln Gln Gly Asn Val Phe Ser Cys Ser Val Met His Glu Ala Leu His
        195                 200                 205
Asn His Tyr Thr Gln Lys Ser Leu Ser Leu Ser Pro Gly
    210                 215                 220
<210> 8
<211> 214
<212> PRT
<213> Artificial Sequence
<220>
<223> Second polypeptide of anti-CD47/PDL1 bispecific antibody
<400> 8
Asp Ile Gln Met Thr Gln Ser Pro Ser Ser Val Ser Ala Ser Val Gly
1               5                   10                  15
Asp Arg Val Thr Ile Thr Cys Arg Ala Ser Gln Gly Ile Ser Arg Trp
            20                  25                  30
Leu Ala Trp Tyr Gln Gln Lys Pro Gly Lys Ala Pro Lys Leu Leu Ile
        35                  40                  45

Tyr Ala Ala Ser Ser Leu Gln Ser Gly Val Pro Ser Arg Phe Ser Gly
    50                  55                  60
Ser Gly Ser Gly Thr Asp Phe Thr Leu Thr Ile Ser Ser Leu Gln Pro
65                  70                  75                  80
Glu Asp Phe Ala Thr Tyr Tyr Cys Gln Gln Thr Val Ser Phe Pro Ile
                85                  90                  95
Thr Phe Gly Gly Gly Thr Lys Val Glu Ile Lys Arg Thr Val Ala Ala
            100                 105                 110
Pro Ser Val Phe Ile Phe Pro Pro Ser Asp Glu Gln Leu Lys Ser Gly
        115                 120                 125
Thr Ala Ser Val Val Cys Leu Leu Asn Asn Phe Tyr Pro Arg Glu Ala
    130                 135                 140
Lys Val Gln Trp Lys Val Asp Asn Ala Leu Gln Ser Gly Asn Ser Gln
145                 150                 155                 160
Glu Ser Val Thr Glu Gln Asp Ser Lys Asp Ser Thr Tyr Ser Leu Ser
                165                 170                 175
Ser Thr Leu Thr Leu Ser Lys Ala Asp Tyr Glu Lys His Lys Val Tyr
            180                 185                 190
Ala Cys Glu Val Thr His Gln Gly Leu Ser Ser Pro Val Thr Lys Ser
        195                 200                 205
Phe Asn Arg Gly Glu Cys
    210
<210>  9
<211>  107
<212>  PRT
<213>  Artificial Sequence
<220>
<223>  VL amino acid sequence of anti-CD47 antibody ADI-29341
<400>  9
Asp Ile Gln Met Thr Gln Ser Pro Ser Ser Val Ser Ala Ser Val Gly
1               5                   10                  15
Asp Arg Val Thr Ile Thr Cys Arg Ala Ser Gln Gly Ile Ser Arg Trp
            20                  25                  30
Leu Ala Trp Tyr Gln Gln Lys Pro Gly Lys Ala Pro Lys Leu Leu Ile
        35                  40                  45
Tyr Ala Ala Ser Ser Leu Gln Ser Gly Val Pro Ser Arg Phe Ser Gly
    50                  55                  60
Ser Gly Ser Gly Thr Asp Phe Thr Leu Thr Ile Ser Ser Leu Gln Pro
65                  70                  75                  80
Glu Asp Phe Ala Thr Tyr Tyr Cys Gln Gln Thr Val Ser Phe Pro Ile
                85                  90                  95
Thr Phe Gly Gly Gly Thr Lys Val Glu Ile Lys
            100                 105
<210>  10
<211>  11
<212>  PRT
<213>  Artificial Sequence
<220>
<223>  VL CDR1 of anti-CD47 antibody ADI-29341
<400>  10
Arg Ala Ser Gln Gly Ile Ser Arg Trp Leu Ala
1               5                   10
<210>  11
<211>  7
<212>  PRT
<213>  Artificial Sequence
<220>
<223>  VL CDR2 of anti-CD47 antibody ADI-29341
<400>  11
Ala Ala Ser Ser Leu Gln Ser
1               5
<210>  12
<211>  9

<212>  PRT
<213>  Artificial Sequence
<220>
<223>  VL CDR3 of anti-CD47 antibody ADI-29341
<400>  12
Gln Gln Thr Val Ser Phe Pro Ile Thr
1               5
<210>  13
<211>  107
<212>  PRT
<213>  Artificial Sequence
<220>
<223>  Amino acid sequence of human kappa light chain constant region (CL)
<400>  13
<210>  14
<211>  510
<212>  PRT
<213>  Artificial Sequence
<220>
<223>  Third peptide chain of bispecific antibody (with linker peptide
between VHHs)
<400>  14
Gln Val Gln Leu Gln Glu Ser Gly Gly Gly Leu Val Gln Pro Gly Gly
1               5                   10                  15
Ser Leu Arg Leu Ser Cys Ala Ala Ser Ala Tyr Thr Ile Ser Arg Asn
            20                  25                  30
Ser Met Gly Trp Phe Arg Gln Ala Pro Gly Lys Gly Leu Glu Gly Val
        35                  40                  45
Ala Ala Ile Glu Ser Asp Gly Ser Thr Ser Tyr Ser Asp Ser Val Lys
    50                  55                  60
Gly Arg Phe Thr Ile Ser Leu Asp Asn Ser Lys Asn Thr Leu Tyr Leu
65                  70                  75                  80
Glu Met Asn Ser Leu Arg Ala Glu Asp Thr Ala Val Tyr Tyr Cys Ala
                85                  90                  95
Ala Pro Lys Val Gly Leu Gly Pro Arg Thr Ala Leu Gly His Leu Ala
            100                 105                 110
Phe Met Thr Leu Pro Ala Leu Asn Tyr Trp Gly Gln Gly Thr Leu Val
        115                 120                 125
Thr Val Ser Ser Gly Gly Gly Gly Ser Gly Gly Gly Gly Ser Gly Gly
    130                 135                 140
Gly Gly Ser Gly Gly Gly Gly Ser Gln Val Gln Leu Gln Glu Ser Gly
145                 150                 155                 160
Gly Gly Leu Val Gln Pro Gly Gly Ser Leu Arg Leu Ser Cys Ala Ala
                165                 170                 175
Ser Ala Tyr Thr Ile Ser Arg Asn Ser Met Gly Trp Phe Arg Gln Ala
            180                 185                 190
Pro Gly Lys Gly Leu Glu Gly Val Ala Ala Ile Glu Ser Asp Gly Ser
        195                 200                 205
Thr Ser Tyr Ser Asp Ser Val Lys Gly Arg Phe Thr Ile Ser Leu Asp

210                 215                 220
Asn Ser Lys Asn Thr Leu Tyr Leu Glu Met Asn Ser Leu Arg Ala Glu
225                 230                 235                 240
Asp Thr Ala Val Tyr Tyr Cys Ala Ala Pro Lys Val Gly Leu Gly Pro
                245                 250                 255
Arg Thr Ala Leu Gly His Leu Ala Phe Met Thr Leu Pro Ala Leu Asn
            260                 265                 270
Tyr Trp Gly Gln Gly Thr Leu Val Thr Val Ser Ser Asp Lys Thr His
        275                 280                 285
Thr Cys Pro Pro Cys Pro Ala Pro Glu Ala Ala Gly Gly Pro Ser Val
    290                 295                 300
Phe Leu Phe Pro Pro Lys Pro Lys Asp Thr Leu Met Ile Ser Arg Thr
305                 310                 315                 320
Pro Glu Val Thr Cys Val Val Val Asp Val Ser His Glu Asp Pro Glu
                325                 330                 335
Val Lys Phe Asn Trp Tyr Val Asp Gly Val Glu Val His Asn Ala Lys
            340                 345                 350
Thr Lys Pro Arg Glu Glu Gln Tyr Asn Ser Thr Tyr Arg Val Val Ser
        355                 360                 365
Val Leu Thr Val Leu His Gln Asp Trp Leu Asn Gly Lys Glu Tyr Lys
    370                 375                 380
Cys Lys Val Ser Asn Lys Ala Leu Pro Ala Pro Ile Glu Lys Thr Ile
385                 390                 395                 400
Ser Lys Ala Lys Gly Gln Pro Arg Glu Pro Gln Val Cys Thr Leu Pro
                405                 410                 415
Pro Ser Arg Asp Glu Leu Thr Lys Asn Gln Val Ser Leu Ser Cys Ala
            420                 425                 430
Val Lys Gly Phe Tyr Pro Ser Asp Ile Ala Val Glu Trp Glu Ser Asn
        435                 440                 445
Gly Gln Pro Glu Asn Asn Tyr Lys Thr Thr Pro Pro Val Leu Asp Ser
    450                 455                 460
Asp Gly Ser Phe Phe Leu Val Ser Lys Leu Thr Val Asp Lys Ser Arg
465                 470                 475                 480
Trp Gln Gln Gly Asn Val Phe Ser Cys Ser Val Met His Glu Ala Leu
                485                 490                 495
His Asn His Tyr Thr Gln Lys Ser Leu Ser Leu Ser Pro Gly
            500                 505                 510
<210>  15
<211>  132
<212>  PRT
<213>  Camelus Linnaeus
<220>
<223>  Camel VHH
<400>  15
Gln Val Gln Leu Gln Glu Ser Gly Gly Gly Ser Val Gln Ala Gly Gly
1               5                   10                  15
Ser Leu Arg Leu Ser Cys Gln Ala Ser Ala Tyr Thr Ile Ser Arg Asn
            20                  25                  30
Ser Met Gly Trp Phe Arg Gln Ala Pro Gly Lys Gln Arg Glu Gly Val
        35                  40                  45
Ala Ala Ile Glu Ser Asp Gly Ser Thr Ser Tyr Ser Asp Ser Val Lys
    50                  55                  60
Gly Arg Phe Thr Ile Ser Leu Gly Asn Ala Lys Asn Thr Leu Tyr Leu
65                  70                  75                  80
Glu Met Asn Ser Leu Lys Pro Glu Asp Thr Ala Met Tyr Tyr Cys Ala
                85                  90                  95
Ala Pro Lys Val Gly Leu Gly Pro Arg Thr Ala Leu Gly His Leu Ala
            100                 105                 110
Phe Met Thr Leu Pro Ala Leu Asn Tyr Trp Gly Gln Gly Thr Gln Val
        115                 120                 125
Thr Val Ser Ser
    130
<210>  16

<211>  132
<212>  PRT
<213>  Artificial Sequence
<220>
<223>  Humanized VHH
<400>  16
Gln Val Gln Leu Gln Glu Ser Gly Gly Gly Leu Val Gln Pro Gly Gly
1               5                   10                  15
Ser Leu Arg Leu Ser Cys Ala Ala Ser Ala Tyr Thr Ile Ser Arg Asn
            20                  25                  30
Ser Met Gly Trp Phe Arg Gln Ala Pro Gly Lys Gly Leu Glu Gly Val
        35                  40                  45
Ala Ala Ile Glu Ser Asp Gly Ser Thr Ser Tyr Ser Asp Ser Val Lys
    50                  55                  60
Gly Arg Phe Thr Ile Ser Leu Asp Asn Ser Lys Asn Thr Leu Tyr Leu
65                  70                  75                  80
Glu Met Asn Ser Leu Arg Ala Glu Asp Thr Ala Val Tyr Tyr Cys Ala
                85                  90                  95
Ala Pro Lys Val Gly Leu Gly Pro Arg Thr Ala Leu Gly His Leu Ala
            100                 105                 110
Phe Met Thr Leu Pro Ala Leu Asn Tyr Trp Gly Gln Gly Thr Leu Val
        115                 120                 125
Thr Val Ser Ser
    130
<210>  17
<211>  10
<212>  PRT
<213>  Artificial Sequence
<220>
<223>  CDR1 of VHH
<400>  17
Ala Tyr Thr Ile Ser Arg Asn Ser Met Gly
1               5                   10
<210>  18
<211>  7
<212>  PRT
<213>  Artificial Sequence
<220>
<223>  CDR2 of VHH
<400>  18
Ile Glu Ser Asp Gly Ser Thr
1               5
<210>  19
<211>  26
<212>  PRT
<213>  Artificial Sequence
<220>
<223>  CDR3 of VHH
<400>  19
Ala Ala Pro Lys Val Gly Leu Gly Pro Arg Thr Ala Leu Gly His Leu
1               5                   10                  15
Ala Phe Met Thr Leu Pro Ala Leu Asn Tyr
            20                  25
<210>  20
<211>  20
<212>  PRT
<213>  Artificial Sequence
<220>
<223>  Linker peptide
<400>  20
Gly Gly Gly Gly Ser Gly Gly Gly Gly Ser Gly Gly Gly Gly Ser Gly
1               5                   10                  15
Gly Gly Gly Ser

            20
<210>  21
<211>  226
<212>  PRT
<213>  Artificial Sequence
<220>
<223>  Fc region amino acid sequence derived from human IgG1
<400>  21
Asp Lys Thr His Thr Cys Pro Pro Cys Pro Ala Pro Glu Ala Ala Gly
1               5                   10                  15
Gly Pro Ser Val Phe Leu Phe Pro Pro Lys Pro Lys Asp Thr Leu Met
            20                  25                  30
Ile Ser Arg Thr Pro Glu Val Thr Cys Val Val Val Asp Val Ser His
        35                  40                  45
Glu Asp Pro Glu Val Lys Phe Asn Trp Tyr Val Asp Gly Val Glu Val
    50                  55                  60
His Asn Ala Lys Thr Lys Pro Arg Glu Glu Gln Tyr Asn Ser Thr Tyr
65                  70                  75                  80
Arg Val Val Ser Val Leu Thr Val Leu His Gln Asp Trp Leu Asn Gly
                85                  90                  95
Lys Glu Tyr Lys Cys Lys Val Ser Asn Lys Ala Leu Pro Ala Pro Ile
            100                 105                 110
Glu Lys Thr Ile Ser Lys Ala Lys Gly Gln Pro Arg Glu Pro Gln Val
        115                 120                 125
Cys Thr Leu Pro Pro Ser Arg Asp Glu Leu Thr Lys Asn Gln Val Ser
    130                 135                 140
Leu Ser Cys Ala Val Lys Gly Phe Tyr Pro Ser Asp Ile Ala Val Glu
145                 150                 155                 160
Trp Glu Ser Asn Gly Gln Pro Glu Asn Asn Tyr Lys Thr Thr Pro Pro
                165                 170                 175
Val Leu Asp Ser Asp Gly Ser Phe Phe Leu Val Ser Lys Leu Thr Val
            180                 185                 190
Asp Lys Ser Arg Trp Gln Gln Gly Asn Val Phe Ser Cys Ser Val Met
        195                 200                 205
His Glu Ala Leu His Asn His Tyr Thr Gln Lys Ser Leu Ser Leu Ser
    210                 215                 220
Pro Gly
225
<210>  22
<211>  490
<212>  PRT
<213>  Artificial Sequence
<220>
<223>  Third peptide chain of bispecific antibody (without linker peptide
between VHHs)
<400>  22
Gln Val Gln Leu Gln Glu Ser Gly Gly Gly Leu Val Gln Pro Gly Gly
1               5                   10                  15
Ser Leu Arg Leu Ser Cys Ala Ala Ser Ala Tyr Thr Ile Ser Arg Asn
            20                  25                  30
Ser Met Gly Trp Phe Arg Gln Ala Pro Gly Lys Gly Leu Glu Gly Val
        35                  40                  45
Ala Ala Ile Glu Ser Asp Gly Ser Thr Ser Tyr Ser Asp Ser Val Lys
    50                  55                  60
Gly Arg Phe Thr Ile Ser Leu Asp Asn Ser Lys Asn Thr Leu Tyr Leu
65                  70                  75                  80
Glu Met Asn Ser Leu Arg Ala Glu Asp Thr Ala Val Tyr Tyr Cys Ala
                85                  90                  95
Ala Pro Lys Val Gly Leu Gly Pro Arg Thr Ala Leu Gly His Leu Ala
            100                 105                 110
Phe Met Thr Leu Pro Ala Leu Asn Tyr Trp Gly Gln Gly Thr Leu Val
        115                 120                 125
Thr Val Ser Ser Gln Val Gln Leu Gln Glu Ser Gly Gly Gly Leu Val

130                 135                 140
Gln Pro Gly Gly Ser Leu Arg Leu Ser Cys Ala Ala Ser Ala Tyr Thr
145                 150                 155                 160
Ile Ser Arg Asn Ser Met Gly Trp Phe Arg Gln Ala Pro Gly Lys Gly
                165                 170                 175
Leu Glu Gly Val Ala Ala Ile Glu Ser Asp Gly Ser Thr Ser Tyr Ser
            180                 185                 190
Asp Ser Val Lys Gly Arg Phe Thr Ile Ser Leu Asp Asn Ser Lys Asn
        195                 200                 205
Thr Leu Tyr Leu Glu Met Asn Ser Leu Arg Ala Glu Asp Thr Ala Val
    210                 215                 220
Tyr Tyr Cys Ala Ala Pro Lys Val Gly Leu Gly Pro Arg Thr Ala Leu
225                 230                 235                 240
Gly His Leu Ala Phe Met Thr Leu Pro Ala Leu Asn Tyr Trp Gly Gln
                245                 250                 255
Gly Thr Leu Val Thr Val Ser Ser Asp Lys Thr His Thr Cys Pro Pro
            260                 265                 270
Cys Pro Ala Pro Glu Ala Ala Gly Gly Pro Ser Val Phe Leu Phe Pro
        275                 280                 285
Pro Lys Pro Lys Asp Thr Leu Met Ile Ser Arg Thr Pro Glu Val Thr
    290                 295                 300
Cys Val Val Val Asp Val Ser His Glu Asp Pro Glu Val Lys Phe Asn
305                 310                 315                 320
Trp Tyr Val Asp Gly Val Glu Val His Asn Ala Lys Thr Lys Pro Arg
                325                 330                 335
Glu Glu Gln Tyr Asn Ser Thr Tyr Arg Val Val Ser Val Leu Thr Val
            340                 345                 350
Leu His Gln Asp Trp Leu Asn Gly Lys Glu Tyr Lys Cys Lys Val Ser
        355                 360                 365
Asn Lys Ala Leu Pro Ala Pro Ile Glu Lys Thr Ile Ser Lys Ala Lys
    370                 375                 380
Gly Gln Pro Arg Glu Pro Gln Val Cys Thr Leu Pro Pro Ser Arg Asp
385                 390                 395                 400
Glu Leu Thr Lys Asn Gln Val Ser Leu Ser Cys Ala Val Lys Gly Phe
                405                 410                 415
Tyr Pro Ser Asp Ile Ala Val Glu Trp Glu Ser Asn Gly Gln Pro Glu
            420                 425                 430
Asn Asn Tyr Lys Thr Thr Pro Pro Val Leu Asp Ser Asp Gly Ser Phe
        435                 440                 445
Phe Leu Val Ser Lys Leu Thr Val Asp Lys Ser Arg Trp Gln Gln Gly
    450                 455                 460
Asn Val Phe Ser Cys Ser Val Met His Glu Ala Leu His Asn His Tyr
465                 470                 475                 480
Thr Gln Lys Ser Leu Ser Leu Ser Pro Gly
                485                 490